# EUROPEAN PATENT SPECIFICATION

(11) **EP 2 120 937 B1**
(45) Date of publication and mention of the grant of the patent: **15.05.2013**
(21) Application number: 08724245.9
(22) Date of filing: 18.03.2008
(51) Int. Cl.: A61K 31/439, A61K 31/438, A61P 25/00

(54) **A COMPOSITION COMPRISING (R)-SPIRO[L- AZABICYCLO[2.2.2]OCTANE-3,2'(3'H) -FURO[2,3 -B]PYRIDINE (AZD0328) AND ITS USE IN THE TREATMENT OF ALZHEIMER'S DISEASE, ADHD OR COGNITIVE DYSFUNCTION**
ZUSAMMENSETZUNG MIT (R)-SPIRO[L-AZABICYCLO-[2.2.2]-OCTAN-3,2'-(3'H)-FURO-[2,3-B]-PYRIDIN (AZD0328) UND IHRE VERWENDUNG BEI DER BEHANDLUNG DER ALZHEIMER-KRANKHEIT, VON ADHD ODER KOGNITIVER DYSFUNKTION
COMPOSITION CONTENANT DE LA (R)-SPIRO[L- AZABICYCLO[2.2.2]OCTANE-3,2'(3'H) -FURO[2,3 -B]PYRIDINE (AZD0328) ET SON UTILISATION DANS LE TRAITEMENT DE LA MALADIE D'ALZHEIMER, D'UN TROUBLE D'HYPERACTIVITÉ AVEC DÉFICIT DE L'ATTENTION (THADA) OU D'UN DYSFONCTIONNEMENT COGNITIF

(30) Priority: 19.03.2007 US 895504 P
(43) Date of publication of application: 25.11.2009
(73) Proprietor: AstraZeneca AB, 151 85 Södertälje (SE); Yale University, New Haven, CT 06511 (US)
(72) Inventor: CASTNER, Stacy A., New Haven, Connecticut 06511 (US); HUDZIK, Thomas, Wilmington, Delaware 19850-5437 (US); MAIER, Donna L., Wilmington, Delaware 19850-5437 (US); MRZLJAK, Ladislav, Wilmington, Delaware 19850-5437 (US); PISER, Timothy, Wilmington, Delaware 19850-5437 (US); SMITH, Jeff S., Wilmington, Delaware 19850-5437 (US); WIDZOWSKI, Dan, Wilmington, Delaware 19850-5437 (US); WILLIAMS, Graham V., New Haven, Connecticut 06511 (US)
(74) Representative: Hällgren, Christer
(86) International application number: PCT/SE2008/050298
(87) International publication number: WO 2008/115139

(56) References cited:
- WO-A1-99/03859
- WO-A2-2006/101745

## Description

### FIELD OF THE INVENTION

The present invention provides for pharmaceutical compositions for treating certain neurologic conditions, certain psychiatric conditions, and other conditions in which subjects have impaired cognitive function.

### BACKGROUND OF THE INVENTION

Diseases that impair normal cognitive function including psychiatric diseases such as schizophrenia and neurodegenerative diseases such as Alzheimer's disease impact sufferers and their families and engender enormous societal and personal suffering, distress, and cost.

The α₇ subtype of neuronal nicotinic receptor (α₇ NNR) has been considered a promising target for effective therapeutics in several diseases that cause cognitive impairment. For example, Alzheimer's disease (AD) is an invariably fatal neurodegenerative disease characterized by progressive impairment of memory, learning abilities, object recognition, disorientation, and decline in language function. Neurodegeneration in AD patients is characterized by progressive loss of neurons in the basal forebrain that synthesize and release the neurotransmitter acetylcholine (ACh). Acetylcholinesterase (AChE) terminates cholinergic neurotransmission by hydrolysing acetylcholine and treatment with acetylcholinesterase inhibitors (AChEI) provide AD patients with modest symptomatic improvement in cognitive function, most likely by prolonging cholinergic neurotransmission. There is a great medical need for drugs with improved and more sustained symptomatic effect and improved tolerability over AChEI drugs. It has been widely postulated that direct-acting agonists of various cholinergic receptors, including α₇ NNRs, may restore lost cholinergic receptor signaling, and therefore represent attractive potential therapeutic agents superior to AChEI. Such medicines are contemplated to improve quality of life and reduce caregiver burden.

Schizophrenia is characterized by cognitive deficits (impairment of working memory, planning, attention deficits) and so-called positive symptoms (hallucinations, delusions, disordered thought, hostility) or negative symptoms (blunted affect, social withdrawal, poor rapport). Cognitive status, not positive symptoms or negative symptoms, correlate with functional outcome. Available typical and atypical antipsychotic drugs provide effective control of only the positive symptoms.

Extensive clinical and pre-clinical studies have demonstrated that cognitive delicits in schizophrenia (CDS) are consistently associated with dysfunction of the dorsolateral prefrontal cortex (dIPFC). Dysfunction in GABA, glutamate, and dopamine neurotransmission in the PFC represent the leading mechanistic hypotheses of cortical dysfunction and cognitive impairment in schizophrenia. Activating glutamate, GABA, and dopaminergic neurotransmission to influence plasticity in PFC synapses that shape network activity subserving cognitive domains impaired in schizophrenia may normalize dlPFC function in schizophrenic patients thereby normalizing performance of cognitive tasks, improving overall cognitive function, and enhancing social reintegration of these patients. There is a great medical need for drugs that improve cognitive function in schizophrenic patients. It has been widely postulated that direct-acting agonists of various cholinergic receptors, including α₇ NNRs, could normalize GABAergic, glutamatergic, or dopaminergic function in the dlPFC, and thus represent attractive potential therapeutic agents for the treatment of cognitive deficits in schizophrenia.

The α₇ subtype of nicotinic acetylcholine receptors (α₇ receptor) are ligand-gated ion channels implicated in synaptic heteroreceptor modulation of major neurotransmitter systems, synaptic plasticity, and learning and memory. AZD0328 is a potent, selective α₇ receptor agonist that can modulate hippocampal and cortical GABAergic, glutamatergic, and dopaminergic neurotransmission by activating the α₇ receptor.

### DESCRIPTION OF THE INVENTION:

We have discovered that (*R*)-Spiro[1-azabicyclo[2.2.2]octane-3,2'(3'H)-furo[2,3-b]pyridine] having the following structure:

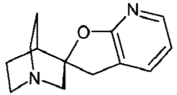

(AZD0328 or AZD0328), when administered at certain doses well below 0.1 mg per kg (milligrams per kilogram animal body weight) to rats, mice and rhesus monkeys, improves performance in some cognitive tests. Thus, compound is expected to provide relief of memory loss and cognitive deficiencies observed in patients with Alzheimer's disease, for the treatment of Attention-Deficit/Hyperactivity Disorder (ADHD), for the treatment of cognitive dysfunction in patients with schizophrenia or for the treatment of cognitive dysfunction and/or to provide an adjunct to current antipsychotic therapies to ameliorate cognitive deficits in patients with schizophrenia when between 0.025 mg and 0.68 mg are administered to patients.

Previously, AZD0328 has been described, for example in US Patent 6,110,914 and counterpart worldwide patents, as an α₇ agonist. In a widely used model of cognitive dysfunction using fimbria fornix transected rats challenged to perform Delayed Non-Match to Position or Differential Reinforcement of Low Rates of Responding tasks, agonistic doses of 1 mg per kg and higher of AZD0328 effectively compensated for the acetylcholine deficiencies of these animals. These doses were associated with plasma concentrations at which AZD0328 acts as an agonist at a₇ receptors *in vitro.* However, lower concentrations were ineffective in these model systems.

We have now discovered that subagonistic concentrations of AZD0328 elicit cognition enhancement in a variety of animal models. An efficacious dose, as measured in different species and by different tests, varies somewhat. For example, in rhesus monkeys (*macaca mulatta*) performing a spatial working memory task, a dose of AZD0328 in the range 0.00048 to 0.016 mg per kg evoked sustained improvement in performance, while higher or lower doses either had no effect or transiently impaired performance. In mice (*mus musculus*) performing Novel Object Recognition (NOR) an episodic memory task, a dose of AZD0328 in the range of 0.00178 to 1.78 mg per kg improved performance. In rats, (*rattus norvegicus*) acquiring operant responding for delayed food reward, a dose of AZD0328 in the range of 0.001 to 1.8 mg per kg, improved performance, while higher and lower doses were ineffective.

These studies, taken together, illustrate that very low doses of AZD0328 unexpectedly enhance cognition and suggest that such low doses will be effective to treat cognitive impairment in human subjects.

While not wishing to be bound by theory, the finding of cognition enhancement at concentrations of AZD0328 well below those able to activate α₇ receptors suggests that the effect is mediated by a novel mechanism.

It is expected that very low doses of AZD0328 as described herein will enhance cognition in the treatment of Alzheimer's disease, in the treatment of Attention-Deficit/Hyperactivity Disorder (ADHD), in the treatment of Cognitive dysfunction in patients with schizophrenia or in the treatment of cognitive dysfunction.

Thus, we provide pharmaceutical compositions for neurological, psychiatric, and other diseases in which cognitive function is impaired. These are used to administer a total unit dose of AZD0328 in the range of 0.025-0.68 mg. .

AZD0328 is an α₇ receptor agonist previously described by AstraZeneca that has now been found to improve performance of cognitive tasks in multiple animal models. The present invention thus arises from the observation that AZD0328 improves cognitive performance in animal models by at surprisingly low doses and plasma concentrations, doses far lower than those used in earlier experiments with this compound, and at far lower plasma concentrations than those that activate the α₇ receptor *in vitro.* AZD0328 is thus expected to provide relief of memory loss and cognitive deficits observed in patients with Alzheimer's disease and if used to provide an adjunct to current antipsychotic therapies to ameliorate cognitive deficits in patients with schizophrenia at surprisingly low doses and plasma concentrations, when administered at doses far below a dose of 0.1 mg per kg of body weight or 2-10 mg total human dose previously described. Particularly, it is expected that treatment of patients with the doses of AZD0328 as described herein will enhance cognition when used for the treatment of Alzheimer's Disease, for the treatment of Attention-Deficit/Hyperactivity Disorder (ADHD), for the treatment of Cognitive dysfunction in patients with Schizophrenia or for the treatment of Cognitive dysfunction.

Accordingly, subjects suffering from conditions characterized by cognitive impairment may be treated with tiny amounts of AZD0328, pharmaceutical compositions may be prepared containing such amounts of AZD0328, and treatment regimens may be developed using AZD0328 to provide lasting cognition enhancement.

The present invention is beneficial because efficacy is achieved with very low doses that achieve plasma concentrations of AZD0328 which are insufficient to cause serious side effects, such as prolongation of the cardiac QT interval, that have been observed at higher doses of AZD0328 in several pre-clinical models. Because use of these lower doses will avoid drug-induced adverse events, administering AZD0328 to improve cognitive function at the doses described herein opens a broad window of safe and effective doses.

Compound I, or AZD0328, is (*R*)-Spiro[1-azabicyclo[2.2.2]octane-3,2'(3'H)-furo[2,3-b]pyridine] as a substantially stereochemically pure enantiomer (configuration is *R*). The compound can exist as a base or a salt thereof and particularly as a D-tartrate monohydrate salt, hereafter referred to as AZD0328 tartrate.

Previously *in vitro* pharmacology studies showed that AZD0328 is an agonist of human, rat, and mouse α₇ receptors, and that it modulates glutamate and GABA synaptic transmission by activating α₇ receptors in rat and monkey brain slices. The potency of AZD0328 to activate α₇ receptors in these *in vitro* preparations was in the micromolar range. Generally, concentrations greater than 100 nM of AZD0328 were necessary to evoke any detectable activation of α₇ receptors, whether human, rat, or mouse recombinant receptors expressed in xenopus oocytes, or in human HEK cells, or native receptors present in acutely dissociated rat hippocampal neurons, or various brain slice preparations. Consistent with these observations, doses of greater than 1 mg per kg were found to be necessary to reverse deficits caused by transection of the fimbria-fornix in rats. For example, 1 or 3 mg/kg of AZD0328 reversed deficits in hippocampal long-term potentiation or response accuracy in two different cognitive tests in fimbria-fornix lesioned animals (delayed non-match to position and differential reinforcement of low rates of responding). The minimum effective dose of 1 mg per kg necessary to reverse deficits in the rat fimbria fornix model produced plasma concentrations in the range 0.29-1.4 µM in the rat, consistent with the range of concentrations required to activate α₇ responses *in vitro.*

We have now discovered that much lower doses of AZD0328 that provide much lower plasma concentrations, can improve performance of cognitive tasks in mice, rats, and monkeys. AZD0328 at low doses increased novel object recognition in mice. In this procedure, mice were habituated to a chamber in which they were then allowed to "sample" two objects before being removed from the chamber. After a delay during which one of the objects was replaced with a novel object, the mice were returned to the chamber for testing. If the delay was brief, mice spent more time exploring the novel object than the familiar object, which is thought to indicate that the mice "recognize" the familiar object, a measure of episodic memory. Under these conditions, mice administered AZD0328 subcutaneously prior to sampling the objects exhibited a higher object recognition index than mice administered vehicle. 0.00178, 0.089, or 1.78 mg/kg sc AZD0328 significantly increased the object recognition index.

AZD0328 at low doses facilitated rat acquisition of operant responding for a delayed food reward. In this procedure, task-naïve, food-restricted rats were placed in a chamber overnight where they could press a bar (respond) to receive a food pellet (reward) after a delay. When the delay between response and reward was long enough to decrease the number of animals responding, rats administered AZD0328 subcutaneously immediately prior to being placed in the chamber responded more often than rats administered vehicle. 0.001, 0.003, 0.01, 0.03, 0.06, 0.6, and 1.8 mg/kg AZD0328 produced a significant increase in the number of rewards earned, indicating that these doses of AZD0328 facilitated acquisition of operant responding, which may be indicative of enhancement of certain aspects of cognition, including but not limited to, attention, spatial exploration, and contingency formation.

AZD0328 at low doses improved performance in the spatial delayed response test in monkeys. Rhesus monkeys were trained in a spatial delayed response task. In this task, food-motivated monkeys observed one of several spatially-displaced wells being baited with a food reward. After a brief delay during which a shutter is drawn and the wells are covered, the monkey must indicate the position of the baited well to receive the food reward. The performance of groups of monkeys was titrated into a narrow range by changing the number of wells or the duration of the delay. Monkeys were tested extensively to establish baseline performance before being administered drug or vehicle. Intramuscular administration of 0.00048 or 0.0016 mg/kg AZD0328 evoked a sustained increase in the percentage of correct responses monkeys made when compared with responses of vehicle-treated monkeys. Notably, 0.016 mg/kg of AZD0328 produced a non-significant increase in the percent correct responses across a group of monkeys, although one monkey in the group exhibited significantly higher percent correct responses when compared with performance at baseline. Higher doses (≥0.016 mg/kg or higher) and lower doses (≤0.00016 mg/kg) had either no effect on monkeys or transiently impaired their performance of the task.

Pharmacokinetic studies in rats and monkeys were used to estimate the plasma concentrations associated with doses at which AZD0328 improved cognitive performance in these species. For example, in rhesus monkeys, two studies were conducted to determine the pharmacokinetic profile of AZD0328 after administration of 0.048 and 1.7 mg per kg AZD0328. These studies demonstrated a linear relationship both between dose and maximum plasma concentrations and between dose and exposure. These data support a reasonable assumption of dose-exposure linearity sufficient to estimate plasma concentrations at much lower doses with some confidence. We thus estimated the peak plasma concentrations in monkeys that received AZD0328 at 0.00048-0.016 mg per kg to be 0.6-18 nM. Similar studies and calculations in rats, when extended to mice, led us to estimate plasma concentrations at 0.001-0.1 mg per kg body weight to be 0.29-100 nM in these species. The present invention concerns those doses of AZD0328 that achieve comparable low concentrations in human subjects in need thereof.

Effective doses of AZD0328 may be determined experimentally by clinical studies that determine exposure relative to dose in humans, and subsequent studies that demonstrate enhanced cognitive function in healthy elderly volunteers in the range of doses that yield plasma concentrations suggested by the animal model studies. Table A shows plasma exposures measured in a group of healthy human volunteers after administration of single oral doses of AZD0328 in the therapeutic range described herein. Total oral doses of AZD0328 in the range 0.025-0.68 mg produced mean plasma concentrations of 0.6-15 nM, consistent with the range of plasma concentrations found to be associated with improved performance in the spatial delayed response test in monkeys. Total oral doses of AZD0328 in the range 0.005-2 mg produced mean plasma concentrations of 0.17-43.9 nM, within the range of plasma concentrations found to be associated with improved performance in rodent models.

**Table A:**

| Geometric mean (%CV) of PK Parameters Following Single Oral Doses of AZD0328 | | | | | | | |
|---|---|---|---|---|---|---|---|
| | Oral Dose | | | | | | |
| Pharmacokinetic parameter (Units) | 0.005 mg | 0.025 mg | 0.075 mg | 0.23 mg | 0.68 mg | 1.35 mg | 2 mg |
| Cₘₐₓ (nmol/L) | 0.17 (24) | 0.6 (12) | 2.1 (19) | 6.4 (31) | 15.0 (10) | 31.0 (26) | 43.9 (17) |
| Tₘₐₓ (h> * | 0.8 | 1.3 | 1.5 | 1.5 | 2.2 | 3.2 | 2.5 |
| AUC0-t (nmol·h/L) | NC | 4.6 (19) | 15.6 (29) | 58.8 (38) | 132.7 (13) | 299.6 (20) | 395.1 (15) |
| AUC (nmol·h/L) | NC | 6.1 (18) | 16.9 (31) | 65.2 (44) | 143.6 (15) | 333.4 (22) | 420.7 (16) |
| t_{1/2}(h) | NC | 6.3 (18) | 5.9 (22) | 6.7 (24) | 6.0 (19) | 6.9 (11) | 5.6 (9) |
| CL/F (L/h) | NC | 19.0 (18) | 20.5 (31) | 16.3 (44) | 21.9 (15) | 18.7 (22) | 22.0 (16) |
| Cₘₐₓ/Dose | 0.007 (24) | 0.006 (12) | 0.006 (19) | 0.006 (31) | 0.005 (10) | 0.005 (26) | 0.005 (17) |
| AUC/Dose | NC | 0.053 (18) | 0.049 (31) | 0.061 (44) | 0.046 (15) | 0.054 (22) | 0.046 (16) |

| | | | | | | | |
|---|---|---|---|---|---|---|---|
| * - Median NC - Not Calculated | | | | | | | |

It is expected that subjects suffering with Alzheimer's Disease, Attention-Deficit/Hyperactivity Disorder (ADHD), Cognitive dysfunction in association with Schizophrenia or Cognitive dysfunction and in need of treatment therefore will be benefited by treatment with amounts of AZD0328 from 0.025-0.68 mg per unit dose are expected to benefit subjects and to provide cognition enhancement for subjects suffering with Alzheimer's Disease, Attention-Deficit/Hyperactivity Disorder (ADHD), Cognitive dysfunction in association with Schizophrenia or Cognitive dysfunction.

### Examples:

Example 1. A subject suffering from a neurological or psychiatric condition may be treated by administering to a subject in need thereof, at least one therapeutically effective dose in the range of 0.025 milligram to 0.68 milligram of (R)-spiro[1-azabicyclo[2.2.2]octane-3,2'(3'H)-furo[2,3-b]pyridine].

Example 2. The neurological or psychiatric condition mentioned in Example 1 may be selected from Alzheimer's Disease, Attention-Deficit/Hyperactivity Disorder (ADHD), Cognitive dysfunction in association with Schizophrenia or Cognitive dysfunction. Example 3. Relief of memory loss or cognitive deficiency in subjects described in Example 2 may be achieved by treating them as described in Example 1.

Example 4. Relief of memory loss or cognitive deficiency may be provided in a subject with Alzheimer's Disease, Attention-Deficit/Hyperactivity Disorder (ADHD), Cognitive dysfunction in association with Schizophrenia or Cognitive dysfunction, by administering to a patient in need thereof, at least one therapeutically effective dose in the range of 0.025 milligram to 0.68 milligram of (*R*)-spiro[1-azabicyclo[2.2.2]octane-3,2'(3'H)-furo[2,3-b]pyridine].

Example 5. Cognitive deficits in patients with schizophrenia may be ameliorated by administering to a patient in need thereof, at least one therapeutically effective dose in the range or 0.025 milligram to 0.68 milligram of (*R*)-spiro[1-azabicyclo[2.2.2]octane-3,2'(3'H)-furo[2,3-b]pyridine].

Example 6. Example 1 through 5 may be carried out then the therapeutically effective dose of (*R*)-spiro[1-azabicyclo[2.2.2]oetane-3,2'(3'H)-furo[2,3-b]pyridine] is sufficient to provide a Cmax plasma level of between about 0.17 and about 44 nanomolar.

Example 7. More particularly, Examples 1 through 5 may be carried out when the therapeutically effective dose is sufficient to provide a Cmax plasma level of between about 0.6 and about 15 nanomolar.

Example 8. Cognitive deficits in subjects with schizophrenia may be ameliorated by identifying subjects with schizophrenia and administering to said subjects at least one therapeutically effective dose of (*R*)-spiro[1-azabicyclo[2.2.2]octane-3,2'(3'H)-furo[2,3-b]pyridine] where the dose is in the range of 0.025 milligram to 0.68 milligram. Example 9. Example 8 may be carried out by additionally determining the duration of the amelioration of cognitive deficits after a first dose and administering additional therapeutically effective doses of (*R*)-spiro[1-azabicyclo[2.2.2]octane-3,2'(3'H)-furo[2,3-b]pyridine] at intervals sufficient to maintain said amelioration of cognitive deficits. Example 10. Any one of Examples 1 through 9 may be carried out with a therapeutically effective dose selected from about 0.026 mg; about 0.0261 mg to about 0.05 mg; about 0.051 mg to about 0.1 mg; about 0.11 mg to about 0.18 mg; about 0.181 mg to about 0.25 mg; about 0.251 mg to about 0.5; about 0.51 mg to about 0.7 mg;

Example 11. A pharmaceutical composition suitable for carrying out Examples 1 through 10 may be prepared comprising an amount of (*R*)-spiro[1-azabicyclo[2.2.2]octane-3,2'(3'H)-furo[2,3-b]pyridine] between 0.025 milligram and 0.68 milligram per unit dose together with at least one phamiaceutically-acceptable excipient, diluent, or carrier.

Example 12. A pharmaceutical composition may comprise a therapeutically effective amount of (*R*)-spiro[1-azabicyclo[2.2.2]octane-3,2'(3'H)-furo[2,3-b]pyridine] sufficient to provide a Cmax plasma level of between about 0.17 and about 44 nanomolar per unit dose. Example 13. A pharmaceutical composition may comprise a therapeutically effective amount of (*R*)-spiro[1-azabicyclo[2.2.2]octane-3,2'(3'H)-furo[2,3-b]pyridine] sufficient to provide a Cmax plasma level of between about 0.6 and about 15 nanomolar.

Example 14. At least one therapeutically effective dose in the range of 0.025 milligram to 0.68 milligram of (*R*)-spiro[1-azabicyclo[2.2.2]octane-3,2'(3'H)-furo[2,3-b]pyridine] may be used for the therapy of a subject suffering from a neurological or psychiatric condition.

Example 15. The use of 0.025 milligram to 0.68 milligram of (*R*)-spiro[1-azabicyclo[2.2.2]octane-3,2'(3'H)-furo[2,3-b]pyridine] for the therapy of a subject suffering from a neurological or psychiatric condition described herein may be achieved where the neurological or psychiatric condition is selected from Alzheimer's Disease, Attention-Deficit/Hyperactivity Disorder (ADHD), Cognitive dysfunction in association with Schizophrenia or Cognitive dysfunction.

Example 16. 0.025 milligram to 0.68 milligram of (*R*)-spiro[1-azabicyclo[2.2.2]octane-3,2'(3'H)-furo[2,3-b]pyridine] may be used for providing relief of memory loss or cognitive deficiency in subjects in need thereof.

Example 17. At least one therapeutically effective dose in the range of 0.025 milligram to 0.68 milligram of (*R*)-spiro[1-azabicyclo[2.2.2]octane-3,2'(3'H)-furo[2,3-b]pyridine] may be used for therapeutically providing relief of memory loss or cognitive deficiency in a subject with Alzheimer's Disease, Attention-Deficit/Hyperactivity Disorder (ADHD), Cognitive dysfunction in association with Schizophrenia or Cognitive dysfunction.

Example 18. At least one therapeutically effective dose in the range of 0.025 milligram to 0.68 milligram of (*R*)-spiro[1-azabicyclo[2.2.2]octane-3,2'(3'H)-furo[2,3-b]pyridine] may be used for therapeutically ameliorating cognitive deficits in patients with schizophrenia.

Example 19. Any one of Examples 14, 15, 16, 17 or 18 may be carried out by use of a therapeutically effective dose of (*R*)-spiro[1-azabicyclo[2.2.2]octane-3,2'(3'H)-furo[2,3-b]pyridine] sufficient to provide a Cmax plasma level of between about 0.17 and about 44 nanomolar.

Example 20. More particularly, Example 19 may be carried out with a therapeutically effective dose sufficient to provide a Cmax plasma level ofbetween about 0.6 and about 15 nanomolar.

Example 21. The use of (*R*)-spiro[1-azabicyclo[2.2.2]octane-3,2'(3'H)-furo[2,3-b]pyridine] in any of Examples 14, 15, 16, 17, 18, 19 or 20 may be achieved with a therapeutically effective dose selected from about 0.026 mg; about 0.0261 mg to about 0.05 mg; about 0.051 mg to about 0.1 mg; about 0.11 mg to about 0.18 mg; about 0.181 mg to about 0.25 mg; about 0.251 mg to about 0.5; about 0.51 mg to about 0.7 mg

Example 22. (*R*)-Spiro[1-azabicyclo[2.2.2]octane-3,2'(3'H)-furo[2,3-b]pyridine] may also be used in the preparation of a medicament comprising a therapeutically effective dose in the range of 0.025 milligram to 0.68 milligram.

Example 23. (*R*)-Spiro[1-azabicyclo[2.2.2]octane-3,2'(3'H)-furo[2,3-b]pyridine] may be used in the preparation of a medicament where a therapeutically effective dose is selected about 0.026 mg; about 0.0261 mg to about 0.05 mg; about 0.051 mg to about 0.1 mg; about 0.11 mg to about 0.18 mg; about 0.181 mg to about 0.25 mg; about 0.251 mg to about 0.5; about 0.51 mg to about 0.7 mg

Example 24. The methods of treatment may be accomplished with specific weights of (*R*)-Spiro[1-azabicyclo[2.2.2]octane-3,2'(3'H)-furo[2,3-b]pyridine] selected from about 0.026 mg to about 0.7 mg

Example 25. (*R*)-Spiro[1-azabicyclo[2.2.2]octane-3,2'(3'H)-furo[2,3-b]pyridine] may be used in the preparation of a medicament or pharmaceutical composition where a therapeutically effective dose is selected from 0.025 to 0.68 mg. Particularly, therapeutically effective doses of (*R*)-Spiro[1-azabicyclo[2.2.2]octane-3,2'(3'H)-furo[2,3-b]pyridine] may be specific weights selected from about 0.026 mg to about 0.7 mg

### 1. INVESTIGATOR'S BROCHURE

This Application describes the non-clinical data supporting the planned first time into man (FTIM) studies, which aim to investigate the pharmacokinetics (PK), safety, tolerability and pharmacological effect of AZD0328.

### 1.1 Physical, chemical and pharmaceutical properties and formulations

The key aspects of the physical, chemical and pharmaceutical properties and formulation (see Section 3) are summarised below.
- AZD0328 tartrate is a solid substance with a solubility in water of greater than 25 mg/mL at physiological pH.
- AZD0328 has the chemical structure 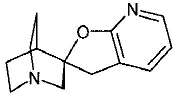
- AZD0328 is available as a powder for oral solution for constitution and dilution.

### 1.2 Non-clinical pharmacology

A summary of the pharmacodynamic properties and safety pharmacology profile of AZD0328 (see Section 4.1) is given below.

### Primary pharmacodynamics

AZD0328 is a reversible, high affinity ligand of human recombinant and rat brain α₇ receptors. In membranes prepared either from cultured, human recombinant α₇ receptor-expressing, human embryonic kidney cells (α₇-HEK) or from rat brain hippocampus, AZD0328 competed with binding of an iodinated form of the high-affinity, selective α₇ receptor antagonist snake toxin, [¹²⁵I]-α-bungarotoxin (BTX). Systemic administration of AZD0328 occupied rat brain α₇ receptors and increased the number of α₇ receptor binding sites. Very low doses of AZD0328 (0.00048-0.0016 mg/kg) improved spatial working memory performance in rhesus monkeys. The pharmacological profile of AZD0328 illustrates its potential utility to improve cognitive function by activating the α₇ receptor.

### Secondary pharmacodynamics

The selectivity of AZD0328 was investigated *in vitro* on 129 molecular targets other than the α₇ nicotinic acetylcholine receptor. AZD0328 inhibited specific radioligand binding at central nicotinic, 5-HT₃ serotonin, α_{2B} norepinephrine, and M₃ and M₅ muscarinic acetylcholine receptors.

### Safety pharmacology

In the core battery and supplemental safety pharmacology studies there were no major findings of concern. The behavioural observation test, modified Irwin screen, in rats showed that below 64.9 mg/kg (300 µmol/kg) there were no significant findings nor any pro- or anticonvulsant properties as tested in the pentylenetetrazol-induced seizure test. In the telemetry study in dogs, iv infusion of AZD0328 showed effects on blood pressure, heart rate and ECG variables (including QT interval, prolongation effect >10% already at the lowest dose). No respiratory effects of AZD0328 were found in rats. At oral doses of 28.1mg/kg (130 µmol/kg) and above inhibited small- and large-intestinal motility in rats in a dose-dependent manner. In conclusion, although many adverse effects are present at exposures in the micromolar range, these are not considered to represent a significant safety concern for patients since effective therapeutic plasma concentrations are expected in the low nanomolar range.

### 1.3 Pharmacokinetics and drug metabolism in animals

### A summary of the non-clinical pharmacokinetic properties of AZD0328 is given below (see Section 4.2)

### Absorption

The PK and toxicokinetic (TK) properties of AZD0328 have been investigated in rat, ferret, mini-pig, rabbit, dog and rhesus monkey. Rapid absorption of AZD0328 in the gastrointestinal tracts was observed in all species, except mini-pig. Elimination half-life was not well characterized across species, but a less than 5 hour elimination half-life was observed with rabbit and ferret. Generally speaking, elimination was completed within the 24 hour interval with the dose levels tested. In the one-month treatment, time and dose dependent PK was not observed in dogs. Significant cerebrospinal fluid (CSF) and brain exposure were demonstrated in rats and rabbits

### Distribution

AZD0328 is a low protein binding molecule across species including human. Following a single oral dose, the maximum blood and plasma concentrations of [¹⁴C]-AZD0328 in all animals were observed at 0.5 h postdose. [¹⁴C]-AZD0328-derived radioactivity was rapidly distributed to the organs and tissues with high levels of radioactivity associated with the contents of the GI tract and the major excretion organs, including kidney and liver. [¹⁴C]-AZD0328-derived radioactivity crossed the placenta.

### Metabolism

The quinuclidinyl-*N*-oxide of AZD0328 was a major metabolite detected in all incubations (including mini-pig microsomes) except for human hepatocytes. Pyridinyl methylation of AZD0328 was a unique polar metabolite (M2) observed in dog hepatocytes. Other minor hydroxylation/oxidation and glucuronide conjugation products were detected in monkey, dog, rat, and guinea pig hepatocyte incubations and mini-pig hepatic microsome incubations

### Excretion

The excretion of [¹⁴C]-AZD0328-derived radioactivity in rats was mainly through the kidney. Radioactivity recovered in urine and feces accounted for approximately 85% and 8%, respectively. No gender difference was noted in the study.

### Drug-drug interactions

AZD0328 exhibited minimal inhibitory effect towards CYP1A2, CYP2C9, CYP2C19, CYP2D6 and CYP3As at concentrations up to 21.6 µg/mL (100 µM). AZD0328 did not significantly inhibit digoxin transport in the cell-based assay with only an 11% decrease at 10.8 µg/mL (50 µM) concentration. This suggests that drug-drug interactions caused by these major metabolic enzymes and the transporter are unlikely.

### 1.4 Toxicology

### The key toxicology findings are as follows (see Section 4.3).

- AZD0328 was not genotoxic in the test systems used.
- CNS effects were seen in all species; nausea, vomiting, convulsions.
- In dogs QTcV prolongation was recorded
- In the rat renal vacuolation was seen

### 1.5 Guidance for investigators

At the time of preparation of this IB, the clinical study programme for AZD0328 had not started, thus no clinical data are currently available.

Non-clinical studies have identified renal tubule vacuolation and these form the basis of the determination of maximum systemic exposure limits in man, ie, 1500 nmol h/L and 600 nmol/L (mean AUC and mean Cₘₐₓ).

In conclusion, with the intended starting dose of 0.03 mg/day, with respect to the observed NOAEL dose (2.2 mg/kg) for renal tubule vacuolation it is judged that AZD0328 can be administered to man in carefully monitored clinical trials.

### 2. INTRODUCTION

### 2.1 Drug description and proposed indication

AZD0328 is expected to provide symptomatic relief of memory loss and cognitive deficits observed in patients with AD and/or provide an adjunct to current antipsychotic therapies to ameliorate cognitive deficits in patients with schizophrenia by binding to α₇ receptors located in brain circuits affected in both AD and CDS including the hippocampus, frontal cortex, and ventral tegmental area (VTA).

### 2.2 Purpose of this Application

This IB is intended to facilitate the understanding of AZD0328 and presents information on the chemical and physical properties of the drug formulation (Section 3), nonclinical information (Section 4).

At the time of preparation of the IB, the clinical programme for AZD0328 had not commenced hence no clinical data are currently available, however, guidance is provided for the investigator in Section 0.

### 2.3 Background and rationale for the development of AZD0328

Alzheimer's disease (AD) is an invariably fatal neurodegenerative disease characterized by progressive impairment of learning abilities, object recognition, disorientation, and decline in language function. Neurodegeneration in AD patients is characterized by progressive loss of neurons in the basal forebrain that synthesize and release the neurotransmitter acetylcholine. Treatment with inhibitors of the enzyme acetycholinesterase (AChEI), which terminates cholinergic neurotransmission by hydrolysis of acetylcholine, offers AD patients modest symptomatic improvement in cognitive function, most likely by facilitating the remaining cholinergic neurotransmission. There is a great medical need for drugs with improved and more sustained symptomatic effect and improved tolerability over AChEI drugs. Such medicines will improve quality of life and reduce caregiver burden. It has been widely postulated that direct-acting agonists of various cholinergic receptors, including the α₇ subtype of nicotinic acetylcholine receptors, by restoring lost cholinergic receptor signaling, represent attractive potential therapeutic agents superior to AChEI..

Cognitive deficits (impairment of working memory, planning, attention, etc) are primary symptoms of schizophrenia. Cognitive status, not positive symptoms (hallucinations, delusions, disordered thought, hostility) or negative symptoms (blunted affect, social withdrawal, poor rapport), correlate with functional outcome. All currently available typical and atypical antipsychotic drugs leave patients cognitively impaired despite effective control of positive symptoms. Extensive clinical and pre-clinical studies have demonstrated that cognitive deficits in schizophrenia (CDS) are consistently associated with dysfunction of the dorsolateral prefrontal cortex (dIPFC). Dysfunction in GABA, glutamate, and dopamine neurotransmission in the PFC represent the leading mechanistic hypotheses of cortical dysfunction and cognitive impairment in schizophrenia. Activating glutamate, GABA, and dopaminergic neurotransmission to influence plasticity in PFC synapses that shape network activity subserving cognitive domains impaired in schizophrenia may normalize dIPFC function in schizophrenic patients thereby normalizing performance of cognitive tasks, improving overall cognitive function, and enhancing social reintegration of these patients.

The α₇ subtype of nicotinic acetylcholine receptors (α₇ receptor) are ligand-gated ion channels implicated in synaptic heteroreceptor modulation of major neurotransmitter systems, synaptic plasticity, and learning and memory. AZD0328, is a potent, selective α₇ receptor agonist that modulates hippocampal and cortical GABAergic, glutamatergic, and dopaminergic neurotransmission, facilitates synaptic plasticity, and improves performance of cognitive tasks in multiple animal models.

### 3. PHYSICAL, CHEMICAL, AND PHARMACEUTICAL PROPERTIES AND FORMULATIONS

### 3.1 Drug substance

AZD0328 drug substance is a stereochemically pure enantiomer (configuration is R). The drug substance is presented as the D-tartrate monohydrate salt, hereafter referred to as AZD0328 Tartrate. One crystalline form of AZD0328 Tartrate has been manufactured to date with a melting point of approximately 198 °C.

The equilibrium solubility of AZD0328 Tartrate inaqueous media is greater than 25 mg/mL (free base equivalent) across the physiological pH range, in water and in saline.

### 3.2 Drug product

The drug product is presented as an AZD0328 powder for oral solution for constitution and dilution. AZD0328 Tartrate is supplied as a powder in amber glass bottles. The drug product will be constituted to a solution using Sodium Chloride Solution for Injection, 9 mg/mL. Further dilution with Sodium Chloride for Injection will allow dosing flexibility as dose ranges of 10 µg to 2 mg (free base equivalent) have been targeted for initial clinical studies. A saline constitution vehicle will be used for the placebo product.

### 3.2.1 Storage conditions

The drug product should be stored and used according to the instructions on the label.

### 4. NON-CLINICAL STUDIES

### 4.1 Non-clinical pharmacology

This section summarizes the pharmacodynamic properties and safety pharmacology profile of AZD0328. Details of each study can be found in Table 1, Table 2 and Table 3.

### 4.1.1 Primary pharmacodynamic studies (in vitro)

In membranes prepared either from cultured, human recombinant α₇ receptor-expressing, human embryonic kidney cells (α₇-HEK) or from rat brain hippocampus, AZD0328 competed with binding of an iodinated form of the high-affinity, selective α₇ receptor antagonist snake toxin, [¹²⁵I]-α-bungarotoxin (BTX) (Table 1). In *Xenopus* oocytes injected with human, recombinant α₇ receptor mRNA, AZD0328 activates whole cell currents (Table 2). Consistent with utility as a heteroreceptor modulator of synaptic neurotransmitter release, AZD0328-evoked whole cell current in α₇-HEK cells exhibited activation kinetics comparable to those of whole cell currents evoked by acetylcholine. In acutely dissociated rat hippocampal interneurons, AZD0328 elicits rapidly activated, desensitizing whole-cell current (Table 1), consistent with the known properties of rat neuronal α₇ receptors. In this preparation, AZD0328 activated currents of similar amplitude to those activated by acetylcholine, suggesting it is a full agonist of rat brain α₇ receptors. AZD0328 modulates synaptic function, consistent with the well-known role of α₇ receptors as heteroreceptor modulators of glutamatergic and GABAergic synaptic function.

In several of these *in vitro* and *in situ* preparations, responses activated by AZD0328 were blocked by low nanomolar concentrations of the α₇ receptor antagonists BTX or methyllycaconitine.

**Table 1 Pharmacological Characterization of AZD0328 in vitro and in situ**

| **System** | **α₇ species** | **measure** | **parameter** | **result** | **unit** | **Conc (µM)** | **Study number** |
|---|---|---|---|---|---|---|---|
| α₇-HEK | Human recombinant | BTX binding | Kᵢ | 3.0 | nM | NA | *AZD0328-1* |
| ^{a}HC membranes | rat | BTX binding | Kᵢ | 4.7 | nM | NA | *AZD0328-1* |
| α₇-HEK | human recombinant | ^{b}AZ binding | Kᵢ | 6.4 | nM | NA | *AZD0328-21* |
| oocyte | human recombinant | Whole cell current | EC₅₀ | 1.9 | µM | NA | *AZD0328-3* |
| oocyte | human recombinant | Whole cell current | Maximum response | 70 | ^{c}%lA | 10 | *AZD0328-3* |
| oocyte | rat recombinant | Whole cell current | EC₅₀ | 1.1 | µM | NA | *AZD0328-3* |
| oocyte | rat recombinant | Whole cell current | Maximum response | 87 | %IA | 10 | *AZD0328-3* |
| oocyte | mouse recombinant | Whole cell current | EC₅₀ | 1.0 | µM | NA | *AZD0328-3* |
| oocyte | mouse recombinant | Whole cell current | Maximum response | 83 | %IA | 10 | *AZD0328-3* |
| α₇-HEK | human recombinant | Whole cell current | ^{d}10-90% rise time | 1.5 | ms | NA | *AZD0328-5* |
| HC neuron | rat | Whole cell current | EC₅₀ | 0.4 | µM | NA | *AZD0328-10* |
| HC neuron | rat | Whole cell current | Maximum Response | 97 | %IA | NA | *AZD0328-10* |
| HC slice | rat | fEPSP | PPF | 121 | ^{f}%cont rol P2:P1 | 5 | *AZD0328-8* |
| HC slice | rat | GABA sIPSC | frequency | 251 | %basel ine | 5 | *AZD0328-8* |
| FFX HC slice | rat | fEPSP | LTP | 153 | %basel ine | 10 | *AZD0328-6* |
| HC slice | marmoset | fEPSP | PPF | 123 | %contr ol P2:P1 | 5 | *AZD0328-9* |
| ^{g}PFC slice | rat | GABA sIPSC frequency | Ec₅₀ | 0.7 | µM | NA | *AZD0328-8* |
| PFC slice | rat | GABA sIPSC frequency | Maximum response | 330 | %baseline | NA | *AD0328-8* |

| | | | | | | | |
|---|---|---|---|---|---|---|---|
| ^{a} HC - hippocampal ^{b} [³H]-AZ11637326, see text for detailed properties ^{c} %IA - percent intrinsic activity, the maximum peak response to AZD0328 as a percentage of the maximum response to the full, endogenous agonist acetylcholine ^{d} 10-90% rise time - a measure of the rate of activation of whole cell currents by AZD0328 ^{f} % control P2:P1 - the percent change in the ratio of the amplitude of fEPSPs recorded in response to two rapidly applied stimuli ^{g} PFC - pre-frontal cortex | | | | | | | |

### 4.1.2 Secondary Pharmacodynamics

Secondary effects of AZD0328 on targets other than the α₇ nicotinic acetylcholine receptor (nAChR) were investigated *in vitro.* Selectivity against a broad panel of 129 receptors, ion channels, nuclear hormone receptors, transporters and enzymes was tested using radioligand binding assays (*Report no. GPW003*). At 10 µM AZD0328 displayed >50% inhibition of specific radioligand binding at central nicotinic, serotonergic 5-HT₃, adrenergic α_{2B}, and muscarinic M₃ and M₅ receptors. In functional assays AZD0328 exhibited little or no selectivity over 5-HT₃ receptors. Thus in terms of functional potency at recombinant human receptors *in vitro,* AZD0328 appears to be equipotent at human recombinant α₇ and 5-HT_{3A/B} receptors and to be 14-fold 5-HT_{3A} > α₇ selective.

### 4.1.3 Primary pharmacodynamic studies (In vivo)

AZD0328 is active in nine primary pharmacological tests in three species at various doses administered by multiple routes of administration (Table 2).

### Receptor occupancy

AZD0328 occupied Sprague Dawley rat brain α₇ receptors and increased the number of α₇ receptor binding sites *in vivo* (Table 2). Across the range of doses that occupied rat brain α₇ receptors, AZD0328 also stimulated dopaminergic neurotransmission. AZD0328 elicited a rapid and significant increase in the spontaneous firing rate of putative dopaminergic neurons within the VTA of anesthetized Sprague Dawley rats, when compared to the firing rate measured in rats administered vehicle.

### Novel object recognition

AZD0328 increased novel object recognition in mice (Table 2). In this procedure, mice were habituated to a chamber in which they were then allowed to "sample" two objects before being removed from the chamber. After a delay during which one of the objects was replaced with a novel object, the mice were returned to the chamber for testing. If the delay was brief, mice spent more time exploring the novel object than the familiar object, which is thought to indicate that the mice "recognize" that the novel object is novel. Under these conditions, mice administered AZD0328 sc prior to sampling the objects exhibited a higher object recognition index than mice administered vehicle. 0.00178 mg/kg sc AZD0328 significantly increased the object recognition index when administered up to and including 48 hours prior to object sampling.

### Operant responding delayed food reward

AZD0328 facilitated rat acquisition of operant responding for a delayed food reward (Table 2). In this procedure, task-naïve, food-restricted rats were placed in a chamber overnight where they could press a bar (respond) to receive a food pellet (reward) after a delay. When there was a delay between response and reward that decreased responding, rats administered AZD0328 sc immediately prior to being placed in the chamber responded more often than rats administered vehicle.

### Delayed, non-match to position

AZD0328 reversed impaired performance of a delayed, non-match to position task (DNMTP) in fimbria-fornix transected (FFX) rats (Table 2). Fimbria-fomix transection severs axons ofbasal forebrain neurons that innervate the hippocampus; DNMTP is a hippocampal-dependent task. Intact and FFX rats were administered vehicle or AZD0328 twice daily and tested in DNMTP for 21 consecutive days. FFX rats exhibited a lower (Area under curve) AUC than intact rats, indicating that they made more errors. AZD0328 increased the AUC of FFX rats over that of FFX rats administered vehicle.

### Hippocampal long-term potentiation

AZD0328 reversed a deficit in hippocampal long-term potentiation (LTP) in FFX Sprague Dawley rats (Table 2). FFX rats exhibited a deficit in hippocampal LTP measured *in vivo* as reduced increase in the slope of the excitatory post-synaptic potential (EPSP) after high-frequency stimuli of the schaffer-colfateral/CA1 synapses. Consistent with observations in the hippocampal slice preparation, AZD0328, administered sc increased the EPSP slope in FFX rats after high-frequency stimulation when compared to the EPSP slope measured in FFX rats that exhibited LTP after vehicle administration. 0.3 mg/kg of AZD0328 was ineffective in this test.

### Spatial delayed response

Rhesus monkey were trained in a spatial delayed response task. Middle doses of AZD0328 (0.0016 and 0.00048 mg/kg im) evoked a sustained improvement in rhesus monkey performance, while a higher dose (0.48 mg/kg) and a lower dose (0.000016 mg/kg) transiently impaired performance of the task (Table 2). Intramuscular administration of middle doses AZD0328 significantly increased the percentage of correct responses compared to monkeys administered vehicle. Four of nine monkeys that exhibited improved performance compared to their own baseline performance after receiving a single dose of AZD0328 at middle doses continued to exhibit improved performance for more than one month before eventually returning to baseline performance.

CNS side effects and decreased spatial working memory performance only occurred together in monkeys administered high dose AZD0328. The NOAEL for decreased spatial working memory performance and oral movements in rhesus monkeys was determined to be 0.016 mg/kg. Doses projected to produce therapeutic benefits in human subjects are those doses that were effective in functional assays across three species and were below the NOAEL for adverse effects on cognitive function and general behaviour in rhesus monkeys (<0.016 mg/kg). Doses projected to produce therapeutic benefits in human subjects therefore fall in the range 0.0001-0.01 mg/kg.

**Table 2 Doses of AZD0328 that were active in pharmacological tests in vivo**

| **Test** | **Species** | **Route** | **Dose (mg/kg)** | **^{a}Time (h)** | **Study number** |
|---|---|---|---|---|---|
| CNS Side Effects | rhesus monkey | Im | 0.48, 0.0000016 | 0-0.5 | *AZD0328-11* |
| Spatial Delayed Response (SDR)- decreased correct responses | rhesus monkey | Im | 0.48, ^{b}0.0016, ^{b}0.00048, 0.000016 | 0.5-0.75 | *AZD0328-11* |
| Operant Acquisition - increased responding | rat | Sc | ^{b}0.00 ^{b}0.003, ^{b}0.01, 0.03, 0.06, 0.6, 1.8 | 0-14 | *AZD0328-12* |
| Novel Object Recognition (NOR) - increased index | mouse | Sc | ^{b}0.00178,0.089, 1.78 | 0.5-0.65 | *AZD0328-13* |
| | | | | | *AZD0328-20* |
| [³H]-AZ11637326 receptor binding *in vivo* (increased) | rat | Sc | 0.0001, 0.001, 0.01,0.3, 1 | 0.5-1.5 | *AZD03287-1* |
| | | | | | *AZD0328-21* |
| Cortical dopamine microdialysis - increased concentration | rat | Sc | ^{b}0.00183,0.0183, 0.51, 5.13 | 0-4 | *AZD0328-15* |
| | | | | | *AZD0328-16* |
| ^{b}Ventral Tegmental Area dopamine neuron firing - increased | rat | Iv | 0.00138 | 0.17-0.5 | *AZD0328-14* |
| ^{b} [¹²⁵I]-α-bungarotoxin binding *ex vivo* increased receptor number | rat | Sc | 0.0001 | 2-4 | *AZD0328-18* |
| Hippocampal LTP- increased | rat | Sc | 1,3 | 0.5-1 | *AZD0328-7* |
| DNMTP-increased AUC | rat | Sc | 1,3 | 0.5-1 | *AZD0328-19* |

| | | | | | |
|---|---|---|---|---|---|
| ^{a}Time after drug administration during which measurements were made ^{b}Doses used to estimate the target dose for efficacy in human subjects | | | | | |

### 4.1.4 Safety pharmacology

This section summarizes the nonclinical safety pharmacology studies of AZD0328 conducted in rats, guinea pigs, rabbits, and dogs. These studies have examined the potential for CNS, cardiovascular, respiratory, gastrointestinal, and renal effects of AZD0328. The potential for abuse liability was also evaluated. Details of each study can be found in Table 3.

### 4.1.4.1 Central Nervous System

### Irwin screen in the rat

The effects of AZD0328 were assessed in the modified Irwin screen, a behavioural, neurological and overt toxicity scoring protocol battery designed to detect a wide range of side effects (Table 3). The findings in the Irwin screen serve as a gross indication of CNS-mediated effects. Occasions of nervous behaviour and reduced grip strength were observed at doses up to 64.9 mg/kg, but these effects were considered mild and not dose-limiting. No significant effects on body weight or pupil size were seen at any dose. In conclusion, the NOAEL of AZD0328 in the Irwin screen was determined to 64.9 mg/kg.

### Spontaneous locomotor activity in rats

AZD0328 was investigated for possible effects on spontaneous locomotor activity in rats (n=6/group)(Table 3). Animals were individually placed in locomotor activity boxes for a 90 min test session. Movements within the box were measured by infrared beams, and activity was assessed as ambulatory counts, stereotypic counts, vertical counts, and resting time.

There was no effect of AZD0328 on any parameter at doses up to 129.8 mg/kg.

### Effects on pentylenetetrazole-induced seizures in the rat

AZD0328 was assessed for its ability to influence the threshold to clonic convulsions caused by an intravenous infusion ofpentylenetetrazole (PTZ) in rats (Table 3). AZD0328 at doses up to 64.9 mg/kg had no significant effect on the threshold level, while at 129.8 mg/kg AZD0328 significantly lowered the threshold for PTZ-induced convulsions by 20% (164 µmol/kg of PTZ). This indicates that AZD0328 exhibited proconvulsant activity at the highest dose, and the NOAEL, therefore, was determined to 64.9 mg/kg.

### Nicotine drug discrimination in rats

AZD0328 was tested for its potential to produce nicotine-like stimulus effects after subcutaneous administration in a drug-discrimination procedure (Table 3). The study was performed in rats (n=6) trained to discriminate 0.3 mg/kg (1.9 µmol/kg) of nicotine from vehicle administered by subcutaneous injections. Nicotine-like discriminative effects in rats have been demonstrated to correlate very well with nicotine-discriminative effects in primates and humans and are used to predict to what extent a compound may be expected to produce nicotine-like psychoactive effects in humans. Response rates were not significantly affected by nicotine. AZD0328, at doses up to 30 mg/kg, severely depressed the rate of responding, thus indicating sufficient doses for CNS stimulation. However, animals did not recognize AZD0328 as a nicotine cue at any dose tested, demonstrating lack of nicotine-like stimulus properties in rats, and suggesting such effects are not predicted in humans.

### Reinforcing potential in rhesus monkey after intravenous injection

The abuse liability potential of AZD0328 was investigated in 6 rhesus monkeys (1 male and 5 females) through a self-administration procedure (Table 3). The compound was compared with cocaine and saline for its ability to maintain intravenous self-administration in the monkeys under a lever-press induced drug infusion schedule. No dose of AZD0328 was self administered at a level greater than saline (range 0.2-1.7 infusions per session), indicating that AZD0328 did not display reinforcing properties. The mean total Cₘₐₓ of AZD0328 at the high dose was 36.2±30.3 µM, which suggests that AZD0328 is not likely to have a high liability for abuse at total plasma concentrations up to 36 µM.

### Behavioral effects in monkeys

Prior to testing its effects on cognitive task performance in marmoset monkeys, the tolerability of AZD0328 was assessed. (Table 3). Behaviours noted in individual marmosets during the five minutes immediately following drug administration were salivation, vomiting, scratching, defecation and increased activity. These observations were not analyzed quantitatively, and suggested that AZD0328 was sufficiently well tolerated in marmosets to warrant assessment of affects on cognitive task performance beginning twenty minutes after drug administration, when these behaviours were no longer apparent.

### 4.1.4.2 Cardiovascular system (In vitro)

### Effects on the hERG channel

A study was performed to evaluate the effects of AZD0328 on the α-subunit of the voltage-dependent potassium channel encoded by the human ether-a-go-go-related gene (hERG), using the patch clamp technique (Table 3). The results showed that AZD0328 blocked the hERG-encoded potassium channel with an estimated IC₅₀ value of 32 µM.

### Effects on dog isolated cardiac Purkinje fibres

Effects of AZD0328 on cardiac action potentials were assessed in 4 dog Purkinje fibre preparations. (Table 3). The following action potential parameters were measured at the stimulation frequencies 0.5 and I Hz: action potential duration at 50% (APD₅₀), 70% (APD₇₀) and 90% (APD₉₀) of repolarisation; the maximum velocity of the action potential upstroke (Vₘₐₓ); the action potential amplitude (APA) and the resting membrane potential (RMP). At both stimulation frequencies, AZD0328 caused a concentration-dependent prolongation of APD₅₀, APD₇₀ and APD₉₀, with significant effects at 5.6 µM and above. There was also an indication of proarrhythmic potential of the compound. A 10% increase in APD₉₀ in the isolated dog Purkinje fibre is seen at concentrations between 5.6 and 16.8 µM.

### Proarrhythmic effects in the isolated perfused rabbit heart

Proarrhythmic properties of AZD0328 were investigated in 6 isolated perfused female rabbit hearts according to the Hondeghem model (Table 3). Effects of AZD0328 on action potential duration at 60% ofrepolarisation (APD₆₀), and Triangulation, Reverse-use dependence and Instability (T, R, I) of the action potential, were studied. Also, a "flag" was raised when T, R or I reached a critical threshold level, below which is considered safe, and the total number of flags was presented as increase in percentage flags. The % flags parameter is considered to be the most sensitive measure of proarrhythmia in this *in vitro* model, with a threshold for cause of concern at 2.5%. The % flags increased concentration-dependently above threshold at 9 and 30 µM. Although TRIad elements were present, there were no overt signs ofproarrhythmia such as early-afterdepolarisations (EADs), Torsade de Pointes, or ventricular fibrillation. In this study the no adverse effect level (NOAEL) of AZD0328 for proarrhythmic signals was 3 µM.

### 4.1.4.3 Cardivascular System (In vivo)

### Cardiac repolarisation in the anesthetised guinea pig

Effects of AZD0328 on ventricular repolarisation and intracardiac conduction were investigated in 6 anesthetised and ventilated guinea pigs (Table 3). For the electrophysiological characterisation, monophasic action potentia MAP duration at 90% repolarisation (MAPD90), ventricular conduction velocity (MAP rise time), and atrioventricular (AV) conduction time were recorded during cardiac pacing. AZD0328 had dose-dependent effects on the electrophysiological activities in the guinea pig heart. At the highest dose, resulting in a total plasma concentration of 8.5 µM, MAPD90 and AV conduction time were prolonged by 20% and 13%, respectively. It is concluded that AZD0328 delayed ventricular repolarisation in the anesthetised guinea pig by 10% at a total plasma concentration of 2.4 µM.

### Cardiovascular effects in the rat

Effects of single oral doses of AZD0328 on mean, systolic and diastolic arterial blood pressures, heart rate, body temperature, locomotor activity and respiratory parameters were evaluated in rats by combining whole body plethysmography with telemetry (Table 3). No significant changes in body temperature or locomotor activity were observed at any dose level. The degree of hypertension, however, was similar at the 3 highest doses, which partly can be explained by the high individual variation in plasma exposure at each dose level. The effect on heart rate was, on the other hand, dose-related. In conclusion, the NOAEL (effect <10%) following oral administration of AZD0328 on cardiovascular function in rats was determined to 0.5 µM (total).

### Cardiovascular effects in the dog

The cardiovascular effects of orally administered AZD0328 were assessed dogs implanted with telemetry transmitters (Table 3). The main purpose of the study was to evaluate effects on the QT interval of the ECG during a 7-days dosing period followed by 5 days of recovery, thus enabling assessment of any accumulation of effects and the recovery back to baseline. Systolic, diastolic and mean arterial blood pressure, heart rate, lead 11 ECG and core body temperature were recorded continuously. The PR, QRS and QT intervals of the ECG were measured, and the QT interval was corrected for changes in heart rate using the method of Van de Water (QTcV). In addition, the dogs were observed for clinical and behavioural signs.

Administration of AZD0328 had no effect on body temperature, PR or QRS interval at any dose level over the 7 or 1 day dosing regimes. In conclusion, both dose-dependent and time-dependent prolongations of the QTcV interval were seen following administration of AZD0328, and no dose was free from effects. Also, frequent vomiting was observed following single doses of 17.3 and 34.6 mg/kg. Thus, no NOAEL for AZD0328 could be established in these dogs, and an increase in QTcV of more than 10% was seen even at the lowest total Cₘₐₓ of 0.8 µM.

### 4.1.4.4 Respiratory system

### Respiratory effects in the rat

Effects of AZD0328 on respiratory function were evaluated in the combined cardiovascular/respiratory study in rats (Table 3). Respiratory rate, tidal volume, inspiratory and expiratory times, and peak inspiratory and expiratory flows were measured before dosing.

No significant changes in arterial blood gases were observed in the satellite animals at any dose level or at any time. The NOAEL following oral administration of AZD0328 on ventilatory function in rats was determined to 31.2 µM.

### 4.1.4.5 Renal function

### Renal function in the rat

Conscious rats (n=9/group) placed in metabolism cages were used to investigate whether AZD0328 had any effects on renal excretion of water and electrolytes (Table 3). Oral administration of AZD0328 had no effects on any of the measured parameters when compared to vehicle treated animals. Thus, no adverse effects were found on renal function in rats at doses up to 50 mg/kg.

### 4.1.4.6 Gastrointestinal function

### Intestinal function in the rat

Effects of AZD0328 on small intestinal and colorectal motility were investigated in rats using the charcoal meal test and the glass bead test, respectively (Table 3). In the first part, evaluating intestinal transit, a test meal containing charcoal as a non-absorbable marker was administered to rats. In the second part, effects of oral administration of AZD0328 on colorectal transit time was evaluated.

AZD0328 significantly demonstrated dose-dependent inhibition of intestinal motility in the rat by AZD0328 at oral doses of 28.1 mg/kg and above. Therefore, the NOAEL on intestinal motility in this study was determined to 5.6 mg/kg.

### Emetic potential in ferrets

The emetic potential of AZD0328 was investigated in ferrets (n=6-8/dose) following a single oral dose of vehicle or AZD0328 (Table 3). Videotape recordings were used for observation of the animals up to 4 h after dose for quantitation and timing of emetic behaviours, characterised as retches (abdominal contractions), vomits and wet-dog shakes.

AZD0328 produced retching, vomiting and wet-dog shakes at all doses, although a statistically significant difference from vehicle treatment was only obtained for retches at 5.6 mg/kg. Thus, the emetic responses were present at all doses but not considered to be dose-related.

**Table 3 Summary of safety pharmacology studies**

| **Organ systems evaluated** | **Species/ strain** | **Method of administration** | **Doses^{a} (mg/kg)** | **Noteworthy findings** | **Study number** |
|---|---|---|---|---|---|
| hERG channel | HEK cells | In vitro | 1, 3, 10, 30 and 100 µM | Block of hGRG tail current with an estimated IC₅₀ of 32 µM. | TSZ67 |
| Purkinje fibre | Dog/Beagle | In vitro | 0.56, 5.6, 16.8 and 56 µM | Reverse-use dependence and 10% increase in cardiac action potential duration between 5.6 and 16.9 µM. | 0181SD |
| Isolated heart | Rabbit/NZW | In vitro | 0.3, 0.9, 3, 9 and 30 µM | Proarrhythmic signals at 9 µM and above. | 0083SB |
| CNS/Irwin | Rat/Wistar | Oral gavage | 0, 10.8, 32.4, 64.9 or 129.8 | Sedation was observed after 129.8 mg/kg up to 4 h postdose. No effect at 64.9 mg/kg. | SP-SPG-0418 |
| CNS/Irwin | Rat/Wistar | Oral gavage | 0, 129.8, 194.7 or 259.5 | Sedation, reduced traction response, lacrimation and salivation at 194.7 mg/kg. At 259.5 mg/kg 1 of 6 animals died. | SP-D0190-SPG-0522 |
| CNS/Locomot or activity | Rat/Wistar | Oral gavage | 0, 32.4, 64.9 or 129.8 | No effect on locomotor activity up to 129.8 mg/kg | SP-SPG-0440 |
| CNS/Proconv ulsant | Rat/Wistar | Oral gavage | 0, 21.6, 64.9 or 129.8 | Reduction of the pentylenetetrazol-induced seizure threshold by 20% at the highest dose. There was no effect at 64.9 mg/kg. | SP-SPG-0431 |
| CNS/Drug-discrimination | Rat/Wistar | sc | 0, 1, 3, 10, 17.5 and 30 | Lack of nicotine-like stimulus properties at doses up to 5.4 mg/kg | SP-D0190-SPG-1734 |
| CNS/Self-administration | Monkey/Rhes us | iv infusion | 0, 0.384, 1.15 and 3.84 mg/kg/infusio n | No reinforcing effect seen at any dose. Total plasma Cₘₐₓ 36.2 µM at the highest dose. In the initial dose-finding study, one monkey receiving 34.8 mg/kg as iv bolus died within minutes. | 03.330/4 |
| CNS/Behavioral effects | Monkey/mann oset | Im | 0.03-3 | One animal defecated after 0.01 mg/kg AZD0328. All of the other behaviours (salivation, vomiting, scratching, defecation and increased activity) were observed in marmosets after administration with 0.03-3 mg/kg AZD0328, but not after vehicle administration | *AZD0328-6* |
| Cardiac repolarisation | Guinea pig | iv, cumulative bolus | 0, 0.06, 0.22, 0.65, 2.16 and 6.49 | Dose-dependent delay in ventricular repolarisation with 10% effect at total plasma concentration 2.4 µM | SP-D0190-SPG-0430 |
| Cardiovascula r/Respiratory | Rat/ Wistar | Oral gavage | 0, 1.1, 32.4, 54.1 or 86.5 | In telemetered rats BP and HR increased for up to 6 h postdose. At highest dose (total Cₘₐₓ 31.2 µM), BP increased by 15% and HR by 19%. No CV effect at 1.1 mg/kg (total Cₘₐₓ 0.5 µM). Whole-body plethysmography registration showed no effects on respiration up to total Cₘₐₓ 31.2 µM. No effect on locomotor activity or body temperature at any dose. | 20030191P CR |
| Cardiovascular/ECG | Dog/Beagle | Oral gavage, bid for 7 days | 5.4 bid, 17.3 or 34.6 single doses | Dose-dependent increase in QTc, with effects >10% in low dose group on day 4 (total Cₘₐₓ 1.5 µM) and day 7 (total Cₘₐₓ 0.8 µM). Mid and high dose groups terminated after first dose due to severe emesis. BP and/or HR increased at all doses. | 0279ZD |
| GI transit | Rat/Long-Evans | Oral gavage | 0, 2.8, 5.6, 28.1, 56.2 or 84.4 | Dose-dependent inhibition of small intestinal and colo-rectat motility from 28.1 mg/kg. No effect at 5.6 mg/kg. | WG-01-75 |
| GI/Emetic response | Ferret/Fitch | Oral gavage | 0, 5.6, 16.9 or 56.2 | Emetic responses at all doses. | WG-02-07 |
| Renal | Rat/Long-Evans | Oral gavage | 0, 2, 10, 20 or 50 | No effect on renal function up to 50 mg/kg. | WG-02-19 |

| | | | | | |
|---|---|---|---|---|---|
| ^{a} Single dose unless specified otherwise. | | | | | |

### 4.1.4.7 Discussion and conclusions regarding primary pharmacology

Primary pharmacology studies show that AZD0328 is a high-affnity, potent agonist of α₇ receptors, and modulates glutamate and GABA synaptic transmission. AZD0328 occupies and upregulates rat α₇ receptors, activates rat mesocortical dopaminergic neurotransmission, and evokes sustained improvement in performance of cognitive tasks after administration of single or multiple doses in mice, rats, and monkeys. These effects are observed in the absence of apparent side effects over the dose range of 0.0001-0.01 mg/kg. The NOAEL for decreased spatial working memory performance and for CNS stimulant-like side effects in rhesus monkeys was determined to be 0.016 mg/kg

The safety pharmacology studies show that AZD0328 does not induce any significant adverse effects on CNS function in rats at oral doses up to 64.9 mg/kg (300 µmol/kg).

The potential of AZD0328 to inhibit cardiac repolarisation was demonstrated. Although no NOAEL for QTc prolongation could be established in the dog telemetry study, the homogenous data obtained in all the repolarisation assays, including a NOAEL of 1 µM in the guinea pig MAP assay and a NOAEL of 0.9 µM in the proarrhythmic isolated heart assay, collectively suggest that effects of AZD0328 on cardiac repolarisation is not expected at plasma concentrations at log-units below 1 µM.

Oral doses of AZD0328 induced hypertension and tachycardia in conscious telemetered rats and dogs. In the rat, an increase in blood pressure and heart rate >10% was seen. There were no effects of AZD0328 on any respiratory parameter in rats.

AZD0328 was shown to cause adverse effects on gastrointestinal function. In the ferret emetic potential model, AZD0328 produced emetic responses. This probably reflects the agonistic activity of AZD0328 at the 5-HT₃ receptor, well known for its involvement in emetic reflexes.

In conclusion, the results from the primary and safety pharmacology studies show that CNS stimulant effects, proconvulsive effects, prolongation of QTc interval, increases in blood pressure, and gastrointestinal effects are the major adverse effects of AZD0328. However, these effects are not considered a risk in the clinic due to large safety margins against the predicted free human therapeutic plasma level of 0.5-1.7 nM.

### 4.2 Pharmacokinetics and drug metabolism in animals

This section provides a summary of the pharmacokinetic and metabolism properties of AZD0328 derived from *in vivo* studies. Systemic exposure of AZD0328 was demonstrated in rat, dog, ferret, Gottingen mini-pigs, New Zealand White rabbits, and rhesus monkey. The derived PK/TK parameters from these studies are presented in Table 4 for single dose studies (DMR4, 0619LR, 20030191PCR, DMO16, DMB10, DMW12, 50190, 0190DD and 03.330/1), and are presented in Table 5 multiple dose PK/TK studies (DMR4, 0612AR, DMB15, 50190, 0190DD and 0295AD).

### 4.2.1 Absorption

Rapid absorption of AZD0328 in the gastrointestinal tracts was observed in all species tested, except mini-pig. In the mass balance study conducted in fed rats, a Tmax of 0.5 h to 1h was observed following single and multiple oral doses (solution). The bioavailability of AZD0328 (total radioactivity) in this study was > 90%. These results suggest that AZD0328 was rapidly and well absorbed in rat. Elimination half-life was not well characterized across species, but a less than 5-hour elimination half-life was observed with rabbit and ferret. Generally speaking, the elimination was complete within the 24-hour interval at the dose levels tested. Following one-month treatment, no time or dose dependent PK was observed in dogs. Gender difference in exposure was observed in rats at the doses tested (females > males), but this was not observed in dogs. Significant cerebrospinal fluid (CSF) and brain exposure were demonstrated in rats and rabbits.

| **PK/TK parameters for AZD0328 from single dose studies** | | | | | | | | | | |
|---|---|---|---|---|---|---|---|---|---|---|
| Species/No. Sex/Study No | **Iv** | | | | **Oral** | | | | | |
| | **Dose mg/kg (µmol/kg)** | **Vss L/kg** | **CL L/h/kg** | **T½ h** | **Dose mg/kg (µmol/kg)** | **Cₘₐₓ ng/mL (µM)** | **Tₘₐₓ h** | **AUC ng h/mL (µM h)** | **F %** | **T**½**h** |
| Rat/2F/0619L R | NA | NA | NA | NA | 149 (690) | 4740-11600 (21.9 53.6) | NA | 76600 (354) | NA | NA |
| Rat/3M/0619 LR | NA | NA | NA | NA | 201 (930) | 3440±567 (15.9 ±2.62) | NA | 49750±7590 (230±35) | NA | NA |
| Rat/1M+1F/D MR4 | 1.08 (5) | NC | NC | NC | 2.16 (10) | 251-344 (1.16-1.59) | 0.5-1 | 699-861 (3.23-3.98) | 78-84 | NC |
| Rat/4M/20030 191PCR | NA | NA | NA | NA | 1.08 (5) | 99.9±38.3 (0.462 ±0.177) | 1 | 441±129 (2.04 ±0.60) | NA | NC |
| Rat/4M/20030 191PCR | NA | NA | NA | NA | 32.4 (150) | 2570±2230 (11.9±10.3) | 1 | 26600±9650 (123±45) | NA | NC |
| Rat/4M/20030 191PCR | NA | NA | NA | NA | 54.1 (250) | 4500±1630 (20.8±7.5) | 1 | 37900±6470 (175±30) | NA | NC |
| Rat/4M/20030 191PCR | NA | NA | NA | NA | 86.5 (400) | 6749±3740 (31.2±17.3) | 1 | 90000±4330 (416±20) | NA | NC |
| Rabbit/3F/D MB10^{η} | NA | NA | NA | NA | 2.16 (10) | 589±206 (2.72±0.95) | 0.5-1.12 | 1750±933 (8.10±4.31) | NA | 2.71 ±1.17 |
| Rabbit/3F/D MB10^{η} | NA | NA | NA | NA | 6.49 (30) | 1750±56 (33.9±6.7) | 0.267- 1.08 | 7340 ±1450 (33.9±6.7) | NA | 4.56 ±1.36 |
| Dog/2F+2M/0 190DD^{αf} | NA | NA | NA | NA | 1.08(5) | 19.2±9.8 (0.0890 ±0.0451) | 1-3 | 68.1±28.1 (0.315±0.130) | NA | NC |
| Dog/2F+2M/0 190DD^{α∂} | NA | NA | NA | NA | 1.08(5) | 33.1 ±21.1 (0.153 ±0.097) | 0.5 | 104±34 (0.483 ±0.158) | NA | NC |
| Dog/2F+2M/0 190DD^{αφ} | NA | NA | NA | NA | 1.08(5) | 26.9±13.8 (0.124±0.064) | 0.5-6 | 177±148 (0.818±0.686) | NA | NC |
| Dog/4M/0279 ZD | NA | NA | NA | NA | 17.3(80) | NA | NA | 2530±736 (11.7 ±3.4) | 0.146 ±0.043 | NA |
| Dog/4M/0279 ZD | NA | NA | NA | NA | 34.6(160) | NA | NA | 8280±3440 (38.3 +15.9) | 0.239 ±0.099 | NA |
| Minipig/3M/ DMW12^{γ} | 8.5 (39.3) | 11.1 ±4.2 | 2.13±0.19 | 3.71±1.72 | 16.9(78.1) | 81.3±37.6 (0.376+0.174) | 1-4 | 404±153 (1.87±0.71) | 5.19 ±2.22 | NC |
| Minipig/2M+ 2F/50190^{f} | NA | NA | NA | NA | 73.1(338) | 379±262 (1.75±1.21) | 2-6 | 3140±2000 (14.5±9.31) | NA | NC |
| Minipig/2M+ 2F/50190^{f} | NA | NA | NA | NA | 563(2602) | 1910±1700 (8.84±7.86) | 12 | 18200±3960 (84.2±18.3) | NA | NC |
| Ferret/4M/D MO16 | NA | NA | NA | NA | 10((46) | 590±392 (2.73±1.81) | 0.25-1 | 4000±1140 (18.3±5.2) | NA | 3.98 ±1.66 |
| Rhesus monkey/2M+ 2F/03.330/1 | 0.385 (1.78) | 3.29±2.32 | 1.26±0.91 | 1.76±0.43 | NA | NA | NA | NA | NA | NA |
| Rhesus monkey/2M+ 2F/03.330/1 | 1.15 (5.32) | 2.52±1.64 | 0.853 ±0.562 | 1.96 ±0.63 | NA | NA | NA | NA | NA | NA |
| Rhesus monkey/2M+ 2F/03.330/1 | 3.85(17.8) | 2.85±1.60 | 0.867 ±0.437 | 2.14 ±0.39 | NA | NA | NA | NA | NA | NA |
| Rhesus monkey/3M/ A7AGBU101 0¹ | NA | NA | NA | NA | 0.048 (0.22) | 16.1 ±2.0 (74.6 ±9.2) | 0.25 | 69 ±13 (14.8 ±2.8)* | NA | 0.85 ±0.23 |
| Rhesus monkey/2F+2 M/A7AGBU1 006^{t} | NA | NA | NA | NA | 1.7(8) | 430±59 (1990 t274) | 0.25 | 634±81(2930 ±374)** | NA | 0.97 ±0.12 |

| | | | | | | | | | | |
|---|---|---|---|---|---|---|---|---|---|---|
| α: Dose was equally split into two with 6 hours apart. t: Intramuscular dose. f: Dose in capsules under fasted condition. *: AUC (0-2h) ∂: Dose in solution under fasted condition. **: AUC (0-6h) φ: Dose in capsules under fed condition. NC: Not calculated. γ: Dose in solution under fed condition. NA: Not available. η: Subcutaneous dose. | | | | | | | | | | |

**Table 5 PK/TK parameters for AZD0328 from multiple dose studies***

| **Species/no. Sex/Study no** | **Dose mg/kg (µmol/kg)** | **Treatment Day** | **Tₘₐₓ h** | **Cₘₐₓ ng/mL (µM)** | **AUC ng h/mL (µM h)** | **AUC/dose** |
|---|---|---|---|---|---|---|
| Rat/1F+1M/DMR4 | 2.16(10) | 1 | 0.5-1 | 251-344 (1.16- 1.59) | 699-861 (3.23-3.98) | 0.323-0.398 |
| Rat/1F+1M/DMR4 | 2.16(10) | 7 | 0.5 | 212-324 (0.981- 1.50) | 599-857 (2.77-3.96) | 0.277-0.396 |
| Rat/4M/0612AR | 2.16(10) | 1 | 1 | 189±42 (1.50 ±0.33) | 528±78 (4.18±0.62) | 0.418±0.062 |
| Rat/4F/0612AR | 2.16 (10) | 1 | 1 | 236 ±18 (1.87 ±0.14) | 916±86 (7.25±0.68) | 0.725±0.068 |
| Rat/4M/062AR | 2.16(10) | 30 | 1 | 154±37 (1.22 ±0.29) | 412±106 (3.26±0.84) | 0.326±0.084 |
| Rat/4F/0612AR | 2.16(10) | 30 | 1 | 220±19 (1.74 ±0.15) | 858±169 (6.79±1.34) | 0.679±0.134 |
| Rat/4M/0612AR) | 15.1 (70) | 1 | 1-3 | 769±126 (6.09 ±1.00) | 4230±328 (33.5±2.6) | 0.479±0.037 |
| Rat/4F/0612AR | 15.1 (70) | 1 | 1 | 984±81 (7.79 ±0.64) | 8120±632 (64.3±5.0) | 0.919±0.071 |
| Rat/4M/0612AR | 15.1 (70) | 30 | 1 | 1160±80 (9.21 ±0.63) | 5630±379 (44.6±3.0) | 0.637±0.043 |
| Rat/4F/0612AR | 15.1 (70) | 30 | 1 | 1420±76 (11.2±0.6) | 9600 ±1120 (76.0±8.9) | 1.09±0.13 |
| Rat/4M/0612AR | 64.9 (300) | 1 | 1-6 | 1670±480 (13.2±3.8) | 23900±3160 (189±25) | 0.630±0.083 |
| Rat/4F/0612AR | 64.9 (300) | 1 | 1-3 | 2480±467 (19.6±3.7) | 40400±3660 (320 ±29) | 1.07±0.10 |
| Rat/4M/0612AR | 64.9 (300) | 30 | 1 | 2780±290 (22.0±2.3) | 29300±2150 (232±17) | 0.773±0.057 |
| Rat/4F/0612AR | 64.9 (300) | 30 | 1 | 2930±707 (23.2±5.6) | 42100±5310 (333±42) | 1.11±0.14 |
| Rabbit/3F/DMB15^{η} | 0.115 (0.3) | 18 | 0.317- 0.970 | 24.7±4.7 (0.111 ±0.016) | 50.2±7.9 (0.205 ±0.053) | 0.148±0.038 |
| Rabbit/3F/DMB15^{η} | 0.269 (0.7) | 18 | 0.217- 0.483 | 138±70 (0.583 ±0.247) | 228±41 (0.872 ±0.342) | 0.270±0.106 |
| Rabbit/3F/DMB15^{η} | 0.384 (1) | 18 | 0.250-2.02 | 78.1±16.8 (0.317 ±0.094) | 232±44 (1.01±0.19) | 0.217±40 |
| Rabbit/2F/DMB15^{η} | 1.15 (3) | 18 | 0.3-1 | 224-296 (1.04-1.37 | 636-846 (2.9-3.9) | 0.212-0.282 |
| Rabbit/3F/DMB15^{η} | 3.84 (10) | 18 | 0.483-2.18 | 677±246 (3.13 ±1.14) | 2510±1290 (11.6±6.0) | 0.251±0.129 |
| Minipig/2F+2M/50190^{f} | 73.1 (338) | 1 | 2-6 | 379±262 (1.75 ±1.21) | 3140±2000 (14.5±9.31) | 0.0429 ±0.0275 |
| Minipig/2F+2M/50190^{f} | 73.1 (338) | 7 | 0.5-4 | 794±316 (3.67 ±1.46) | 4610±2110 (21.3±9.75) | 0.0630 ±0.0288 |
| Minipig/2F+2M/50190^{f} | 422 (1951) | 7 | 12 | 1420-1570 (6.57- 7.24) | 25700-27700 (119-128) | 0.0610-0.0656 |
| Dog/2F+2M/0190DD^{αf} | 34.6 (160) | 1 | 1-3 | 694±387 (3.21 ±1.79) | 4430±3010 (20.5±13.9) | 0.128±0.087 |
| Dog/2F+2M/0190DD^{αf} | 34.6 (160) | 7 | 1 | 2120 ±1070 (9.79 ±4.96) | 6970±3050 (32.2±14.1) | 201±0.088 |
| Dog/2F+2M/0190DD^{αω} | 34.6 (160) | 1 | 0.5 | 1130±234 (5.20 ±1.08) | 4890±500 (22.6±2.3) | 0.141±0.014 |
| Dog/2F+2M/0190DD^{α∂} | 34.6 (160) | 7 | 0.5-1 | 1560±921 (7.22 ±4.26) | 7200±3030 (33.3±14.0) | 0.208±0.088 |
| Dog/2F+2M/0190DD^{αφ} | 138 (640) | 1 | 1-6 | 1520±582 (7.04 ±2.69) | 11900±7330 (55.0±33.9) | 0.0859 ±0.0530 |
| Dog/2F+2M/0190DD^{αφ} | 138 (640) | 7 | 0.5-6 | 1360±666 (6.27 ±3.08) | 7160±2860 (33.1±13.2) | 0.0517 ±0.0206 |
| Dog/3F/0295AD^{αφ} | 8.65 (40) | 7 | 1-7 | 302±186 (1.39 ±0.86) | 981±330 (4.53±1.53) | 0.113±0.038 |
| Dog/3F/0295AD^{αφ} | 8.65(40) | 35 | 1-9 | 270±72 (1.25 ±0.33) | 924±145 (4.27±0.67) | 0.107±0.017 |
| Dog/3M/0295AD^{αφ} | 8.65 (40) | 7 | 0.5-7 | 286±12 (1.32 ±0.06) | 868±441 (4.0±2.04) | 0.100±0.051 |
| Dog/3M/0295AD^{αφ} | 8.65 (40) | 35 | 8.5-9 | 373±80 (1.73 ±0.37) | 1250±341 (5.79 ±1.57) | 0.145±0.039 |
| Dog/3F/0295AD^{αφ} | 17.3 (80) | 7 | 0.5-6.5 | 850±540 (3.93 ±2.49) | 2130 ±796 (9.83±3.68) | 0.246±0.092 |
| Dog/3F/0295AD^{αφ} | 17.3(80) | 35 | 1-9 | 439 ±169 (2.03 ±0.78) | 2020 ±420 (9.33±1.94) | 0.233±0.049 |
| Dog/3M/0295AD^{αφ} | 17.3 (80) | 7 | 6.5-7 | 619±223 (2.86 ±1.03) | 1930±411 (8.92±1.90) | 0.223±0.047 |
| Dog/3M/0295AD^{αφ} | 17.3 (80) | 35 | 1-9 | 608±254 (2.81 ±1.18) | 1690±72 (7.85±0.33) | 0.196±0.008 |
| Dog/6F/0295AD^{αφ} | 34.6 (160) | 7 | 0.5-6.5 | 1880 ±1060 (8.69 ±4.90) | 4160±1990 (19.2 ±9.2) | 0.120 0.058 |
| Dog/6F/0295AD^{βφ} | 26.0 (120) | 35 | 1-8.5 | 1190±385 (5.49 ±1.78) | 3460±942 (16.0±4.4) | 0.133±0.036 |
| Dog/6M/0295AD^{αφ} | 34.6 (160) | 7 | 0.5-7 | 1910 ±949 (8.82 ±4.39) | 5260 ±2610 (24.3±12.1) | 0.152±0.075 |
| Dog/6M/0295AD^{βφ} | 26.0(120) | 35 | 0.5-2 | 1760≥699 (8.16 ±3.23) | 4530±752 (20.9±3.5) | 0.174 ±0.029 |

| | | | | | | |
|---|---|---|---|---|---|---|
| α: Dose was equally split into two with 6 hours apart. β: Dose was split into 17.3 and 8.7 mg/kg with 6 hours apart. f: Dose in capsules under fasted condition. ∂: Dose in solution under fasted condition. φ: Dose in capsules under fed condition. η: Subcutaneous dose. *: As appropriate, some results from day 1 are included. | | | | | | |

### 4.2.2 Distribution

AZD0328 is a low protein binding molecule across species including human (< 15%) across the concentration range 21.6 to 21,630 ng/mL (0.1 to 100 µM). In most species, the binding was concentration and gender independent. In a rat QWBA study (oral dose), the maximum blood and plasma concentrations of [¹⁴C]-AZD0328-derived radioactivity in all animals were observed at 0.5 h postdose, which declined rapidly to levels that were below the limit of quantitation (BLQ) at 24 h postdose. [¹⁴C]-AZD0328-derived radioactivity was rapidly distributed to the organs and tissues with high levels of radioactivity associated with the contents of the GI tract and the major excretion organs, including kidney and liver. The radioactivity declined rapidly and by 168 h postdose radioactivity levels were not detectable in most tissues, with the exception of a few ocular tissues and nasal turbinates. The levels of [¹⁴C]-AZD0328-derived radioactivity in the CNS tissues were greater than blood during the early sampling times, suggesting brain penetration. Notable association of [¹⁴C] -AZD0328-related radioactivity with the melanin-containing tissues was observed, with measurable radioactivity still present in the ocular tissues at 216 h postdose. [¹⁴C]-AZD0328-derived radioactivity crossed the placenta.

### 4.2.3 Metabolism

The quinuclidinyl-*N*-oxide of AZD0328 was a major metabolite (pharmacological inactive) detected in all incubations except for human hepatocytes. Pyridinyl methylation of AZD0328 was a unique polar metabolite (M2) observed in dog hepatocytes. Other minor hydroxylation/oxidation and glucuronide conjugation products were detected in monkey, dog, rat, and guinea pig hepatocyte incubations and mini-pig hepatic microsome incubations. Four separate batches of human hepatocytes were tested, and no metabolites were detected from these hepatocyte incubations. Analysis of plasma and urine samples from either orally or iv dosed rats confirmed *N*-oxide product of AZD0328 was a main product in the excretion and circulation. The percentage of AZD0328 *N*-oxide in the plasma from male rats was much higher than from females.

### 4.2.4 Elimination/excretion

Pharmacokinetics and mass balance of [¹⁴C]-AZD0328 were investigated in rats. The excretion of [¹⁴C]-AZD0328-derived radioactivity in rats was mainly through the kidney. Over the course of the study, radioactivity recovered in urine and feces accounted for approximately 85% and 8%, respectively. No gender or administration route differences were noted in the study. Based on recovered urinary radioactivity (urine plus cage rinse) and the exposure, as evaluated by AUC, after oral versus iv administration, AZD0328 was well absorbed (97% and 95% for male and female, respectively).

AZD0328 exhibited minimal inhibitory effect towards CYP1A2, CYP2C9, CYP2C19, CYP2D6 and CYP3As at concentrations up to 21.6 µg/mL (100 µM). AZD0328 did not significantly inhibit Pgp mediated digoxin transport. These data suggest that drug-drug interactions caused by these major metabolic enzymes and the Pgp transporter are unlikely. Using allometric scaling of in vivo data from rat and monkey, 5.9 mL/min/kg and Vdss 2.7 L/kg have been predicted for human systemic clearance and volume of distribution, respectively. The allometric slop factors for scaled clearance and Vdss were 0.74 and 1.07, respectively, in line with standard allometric experience. Assuming one-compartment pharmacokinetic model, the human predicted half-life for AZD0328 was 5.3 h. The 50% bioavailability of AZD0328 in human was conservatively selected based on the observations from rat, dog and monkey.

### 4.2.5 Conclusions regarding pharmacokinetics and drug metabolism in animals

The exposure to AZD0328 in the pharmacology and toxicity species was demonstrated. The PK properties derived from the preclinical species show that this molecule easily gets into and out of the physiological system and has the properties required to achieve the targeted concentrations at the receptor. Based on the in vitro and in vivo PK findings across species, there are no anticipated issues with this molecule for the first time dosing in man.

### 4.3 Toxicology

This section provides a summary of the toxicology studies performed to support the administration of AZD0328 to humans. The toxicology program has included single-dose and multiple-dose studies in multiple species (rat, dog and minipig), genotoxicity studies, and reproductive toxicity studies.

### 4.3.1 Single-dose toxicity studies in rat

The objective of this study was to investigate the minimum lethal and maximum non-lethal dose of AZD0328 following a single oral administration. Single-dose studies were performed in rats oral and intravenous administration. Clinical signs observed were those expected, ie, ataxia, convulsions, and death. No signs of delayed toxicity were seen. The main acute effect seen in animals given AZD0328 was decreased motor activity, noted within an hour after dose in all groups. At higher doses, pilo-erection, half-shut eyes, tremor, respiratory changes, and spasm were noted. Clonic convulsions were noted prior to all preterminal deaths. In surviving animals, the symptoms gradually disappeared and all animals had recovered 23-24 hours after dose.

In conclusion, in this study the minimum lethal dose was identified as 1400 µmol/kg (300mg/kg) in males and 930 µmol/kg (200 mg/kg) in females, respectively, and the maximum non-lethal dose was 930 µmol/kg (200mg/kg) in males and 690 µmol/kg (150 mg/kg) in females, respectively.

### 4.3.2 Maximum Tolerated Dose and 7 days oral toxicity study in the dog

Single doses of 4.5 to 17 mg/kg were administrated to test AZD0328 to 2 male and 2 female dogs. Cmax values of 2.8 - 14 µmol/L were obtained, which is approximately 1000 times higher than anticipated human exposures in the SAD study. Signs of nausea and at the higher doses vomiting was seen. In the 7 days repeated dose study 2 male and 2 female dogs were given 35 mg/kg per day. However, vomiting was the major clinical sign recorded during the study. The ECG recording showed a slight QT prolongation at doses 8.6 mg/kg or more.

### 4.3.3 Maximum Tolerated Dose and 7 days oral toxicity study in the minipig

A dose range finding study was conducted in minipigs, in order to explore this species as a suitable non-rodent for toxicity studies with AZD0328. 4 animals were used in the escalating phase, where doses up to 1500 mg/kg AZD0328 tartrate salt. In the dose range finding (DRF) phase 4 animals were given doses of 130 and 1000 mg/kg. Deaths were seen after a single dose of 1500 mg/kg and repeated doses of 1000 mg/kg. Other signs seen at higher dose levels were vomiting, loss of appetite and increased heart rate. At 130 mg/kg only subdued behaviour was recorded.

### 4.3.4 Repeated-dose toxicity studies in the rat

The objective of this investigation was to study the toxicity of AZD0328 given orally to rats for 1 month. Four groups of rats, each consisting of 10 males and 10 females, were given 0, 2.2, 15 and 65 mg/kg/day (0, 10, 70 and 300 µmol/kg/day) of AZD0328..

In conclusion, oral administration of AZD0328 to rats for one month at the high dose level of 65 mg/kg/day (300 µmol/kg/day), induced ploughing and salivation in the majority of animals and slight decreases in motor-activity in a few females. Slight losses in body weight were recorded prior to the reduction in the high dose level. The only target organ identified by pathology was the kidney where there was a small increase in the kidney weight of both sexes and vacuolation of the tubular cells, most prominently in the collecting ducts of the medulla and papilla.

### 4.3.5 Repeated-dose toxicity studies in the dog

The aim of this study was to investigate the toxicity caused by oral (capsule) administration of AZD0328 for a period of I month in the dog. Groups of 3 male and 3 female Beagle dogs were dosed twice daily for at least 28 days at total daily dose levels of 0, 8.7, 17 or 35 mg/kg/day (0, 40, 80 or 160 µmol/kg/day). Dose-titration for 6 days up to the selected dose levels preceded the 1 month dosing period.

In conclusion, twice daily oral administration of AZD0328 to dogs for one month following dose titration for 6 days resulted in incidences of subdued behaviour, vomiting with muscle twitch and tonic convulsion recorded in a minority of animals. Reversible prolongations of the QTcv interval were observed at dose levels above 26 mg/kg/day (120 µmol/kg/day). The no adverse effect level for this study was a total daily dose of 17 mg/kg/day (80 µmol/kg/day).

### 4.3.6 Genotoxicity

The genotoxic potential of AZD0328 has been evaluated in the Ames test and in the Mouse lymphoma test. AZD0328 was not mutagenic in either of these tests.

### 4.3.7 Conclusions regarding toxicology

The main safety concern as detected in the toxicology studies are:
- the effects on ECG in dogs, mainly QTcV prolongation.
- the effects on the kidney, with a small increase in kidney weight and vacuolation in the tubules.

The present estimate of therapeutic doses yields a Cₘₐₓ < 1 nm. Compared to the data from the repolarisation assays and estimated NOEL exposures regarding the QTcV in dogs, the effects levels are 500-1000 times higher.

The exposure limit in humans based on AZ standard of a 100 fold margin to QT effects would indicate a maximum plasma concentration of 5 nM in human subjects.

The exposure limit in humans, based on the adverse effects seen in kidney in rats, would be an exposure of AUC 1500 nmolh/L or Cmax 600 nmol/L. This corresponds to half the exposure observed at the NOAEL dose (2.2 mg/kg) in the abovementioned 1-month rat toxicity study.

### 5. EFFECTS IN HUMANS

At the time of preparation of this IB, AZD0328 had not been administered to humans.

### 6. SUMMARY OF DATA AND GUIDANCE FOR THE INVESTIGATORS

### 6.1 Non-clinical pharmacology, PK and toxicology

AZD0328 is a high-affinity, potent, and efficacious agonist at human recombinant and rat brain α7 receptors *in vitro,* and evokes sustained improvement in performance of cognitive tasks in mice, rats, and monkeys. The NOAEL for decreased spatial working memory performance and for CNS stimulant-like side effects in rhesus monkeys was determined to be 0.016 mg/kg, a dose that is estimated to achieve a free plasma Cmax of 18 nM in rhesus monkeys. Doses projected to produce therapeutic benefits in human subjects therefore fall in the range 0.0001-0.01 mg/kg. The safety pharmacology studies show a dose- and time-dependent prolongation of the QTc interval was seen. Although no NOAEL for QTc prolongation could be established in the dog, the homogenous data obtained in all the repolarisation assays, collectively suggest that effects of AZD0328 on cardiac repolarisation is not expected at plasma concentrations at log-units below 1 µM

Historically, the potency of AZD0328 in animal primary pharmacology models was discovered in two phases. First, the efficacy of AZD0328 in the 1 to 3 mg/kg range in the fimbria-fornix lesioned rat model was discovered, and safety pharmacology, toxicology, and the marmoset study were conducted based on these doses. After these studies were completed, the efficacy of AZD0328 in the 0.00048-0.0016 mg/kg range in the rhesus monkey spatial delayed response test was discovered and corroborated with several additional studies in mice and rats. As a result, primary pharmacology studies cover a very wide range of doses well below those used in safety pharmacology and toxicology studies.

AZD0328 was rapidly and well absorbed in rat. Elimination half-life was less than 5-hour observed in rabbit and ferret. The elimination was complete within the 24-hour interval at the dose levels tested. Significant cerebrospinal fluid (CSF) and brain exposure were demonstrated. AZD0328 is a low protein binding molecule across species including human (<15%). The quinuclidinyl-*N*-oxide of AZD0328 was a major metabolite (pharmacological inactive) and the excretion of in rats was mainly through the kidney.

The key toxicology findings were QTcV prolongation in dog, nausea, vomiting and convulsions were CNS effects seen in all species and in the rat, renal vacuolation was seen. AZD0328 was not genotoxic in the test systems used.

### 6.2 Need for safety margins, monitoring and precautions associated with the conduct of a clinical study

### 6.2.1 Systemic exposure limits

Given the findings in the 1 month repeated dose toxicology studies in the rats, it is reasonable to set human systemic exposure limits following single dose administration equal to the exposure observed at NOAEL for renal tubule vacuolation. The corresponding human equivalent dose, based on the FDA guidance (via allometric scaling of body surface area) is, 0.35 mg/kg. A dose of 0.03 mg/day is proposed as a starting dose. This dose is selected as it is expected to yield an exposure (Cmax 0.7 nmol/L and AUC 5.5 nmolh/L) in the range that resulted in improved cognitive performance (Cmax 0.5 . 1.7 nmol/L) in a pharmacodynamic study in rhesus monkeys and well below the NOAEL for behavioral side effects (Cmax 18 nmol/L, AUC 28 nmolh/L). An exposure of AUC 1500 nmolh/L or Cmax 600 nmol/L will not be exceeded. This corresponds to half the exposure observed at the NOAEL dose (2.2 mg/kg) in the abovementioned 1-month rat toxicity study.

### 6.2.2 Safety monitoring

QT effects will be closely monitored by continuous digital ECG and telemetry. CNS effects will be monitored by AE open questions: around the expected Cmax, 6 h after dosing, 24 h after dosing and follow-up. Stopping criteria as listed below will apply. If the following occurs, the SRC will decide whether the tested dose level or a lower dose should be re-tested.

### 6.3 Indications to be studied

The objectives of the clinical program are to demonstrate the efficacy and safety of AZD0328 in the treatment of patients with cognitive impairment, ie, AD or CDS.

### 6.4 Treatment for overdose and adverse reactions

There is no specific antidote available for treatment of overdose of AZD0328, and thus, only symptomatic and supportive treatment is available in such cases. It is unlikely that the elimination of AZD0328 can be increased by forced diuresis or alkalinisation/acidification of the urine since the expected volume of distribution suggests that much more than 95% of the body content is outside the plasma compartment. Adverse reactions should be treated symptomatically.

### 6.5 Risk-benefits analysis and conclusion

### General

Progressive loss of neurons in the basal forebrain that synthesize and release acetylcholine is a major feature of AD. Presently available treatment with inhibitors of the enzyme acetycholinesterase (AchEI) offers AD patients only modest symptomatic improvement in cognitive function. It has been widely postulated that direct acting agonists of various cholinergic receptors, including the α7 subtype of nicotinic acetylcholine receptors, by restoring lost cholinergic receptor signalling, represent attractive potential therapeutic agents superior to AChEI. Dysfunction in GABA, glutamate, and dopamine neurotransmission in the PFC represent the leading hypotheses of cortical dysfunction and cognitive impairment in schizophrenia. Activating glutamate, GABA, and dopaminergic neurotransmission to influence plasticity in PFC synapses may normalize dIPFC function in schizophrenic patients thereby normalizing performance of cognitive tasks.

The α7 subtype of nicotinic acetylcholine receptors (α7 receptor) are ligand-gated ion channels implicated in synaptic heteroreceptor modulation of major neurotransmitter systems. α7 receptors are located in brain circuits affected in both AD and CDS. AZD0328, is an orally active potent, selective α7 receptor agonist under development by AstraZeneca that modulates hippocampal and cortical GABAergic, glutamatergic, and dopaminergic neurotransmission, facilitates synaptic plasticity, and improves performance of cognitive tasks in multiple animal models. There is a great medical need for drugs that may offer an improved and more sustained symptomatic effect and improved tolerability over AChEI drugs in AD. Also there is a great medical need for drugs that may improve CDS. AZD0328 may provide symptomatic relief of memory loss and cognitive deficits observed in patients with AD and/or provide an adjunct to current antipsychotic therapies to ameliorate cognitive deficits in patients with schizophrenia. Hence, there are strong reasons to investigate whether AZD0328 is of value in patients with AD and CDS.

Various adverse effects have been noted in the non-clinical safety pharmacology and toxicity studies, eg sedation, seizures, increased pulse rate and blood pressure, reversible renal vacuolation and also QT prolongation. All adverse effects have occurred at exposures in the micromolar range, these are not considered to represent a significant safety concern for patients since effective therapeutic plasma concentrations are expected in the low nanomolar range.

The reversible kidney effects seen in the rats have been used to set the maximum exposure limit. Due to the lack of an ideal safety variable for monitoring early renal dysfunction/toxicity the upper exposure limit has been set to a level corresponding to half the NOAEL observed in the 1-month rat study.

QT prolongation was observed at higher doses in a dog telemetry study. No QT problems are anticipated in the human studies due to the large safety margin but digital ECG recordings will be performed in the first time in man study and close attention will be paid to any QT changes.

Some CNS-stimulant-like side effects of AZD0328 have been observed in studies on the Rhesus monkey. However these effects were transient, and the relevance to man is unclear. The proposed starting dose in the first time in man study is anticipated to give an exposure with an adequate margin to the exposure causing CNS-side effects in the monkeys. Also, CNS effects are easy to monitor clinically by the subjective experience of the study participants and any CNS side effects during dose escalations should therefore be fairly easy to detect.

### 6.6 Conclusion

Given the observed pharmacological and toxicology profile in non-clinical studies, it is judged that AZD0328 can be given to man in carefully monitored clinical trials. It is concluded that single doses of AZD0328 up to the exposure limit (AUC: 1500 nmolh/L or Cₘₐₓ: 600 nmol/L) together with the safety monitoring procedures are expected not to pose any foreseeable risk of inflicting harm or injury. The potential benefits of a future improved treatment of cognitive dysfunction in AD and a treatment that may ameliorate cognitive deficits in patients with schizophrenia are considered to outweigh any foreseeable risks.

## Claims

1. A pharmaceutical composition comprising an amount of (*R*)-spiro[1-azabicyclo[2.2.2]octane-3,2'(3'H)-furo[2,3-b]pyridine] between 0.025 and 0.68 mg per unit dose together with at least one pharmaceutically acceptable excipient, diluent or carrier.

2. The use of (*R*)-spiro[1-azabicyclo[2.2.2]octane-3,2'(3'H)-furo[2,3-b]pyridine] in the preparation of a medicament comprising a unit dose in the range 0.025-0.68 mg for the therapy of a subject suffering from a neurological or psychiatric condition.

3. The use according to claim 2, wherein said neurological or psychiatric condition is selected from Alzheimer's Disease, Attention-Deficit/Hyperactivity Disorder (ADHD), Cognitive dysfunction in association with Schizophrenia or Cognitive dysfunction.

4. The use according to claim 2 or claim 3 for providing relief of memory loss or cognitive deficiency.

## Patentansprüche

1. Pharmazeutische Zusammensetzung, enthaltend 0,025 bis 0,68 mg (*R*)-Spiro[1-azabicyclo[2.2.2]octan-3,2'(3'H)-furo[2,3-b]pyridin] pro Einheitsdosis, zusammen mit mindestens einem pharmazeutisch unbedenklichen Exzipienten, Verdünnungsmittel oder Träger.

2. Verwendung von (*R*)-Spiro[1-azabicyclo[2.2.2]octan-3,2'(3'H)-furo[2,3-b]pyridin] zur Herstellung eines eine Einheitsdosis im Bereich von 0,025-0,68 mg enthaltenden Medikaments zur Therapie eines an einem neurologischen oder psychiatrischen Leiden leidenden Patienten.

3. Verwendung nach Anspruch 2, wobei das neurologische bzw. psychiatrische Leiden aus Alzheimer-Krankheit, Aufmerksamkeitsdefizit-/Hyperaktivitätsstörung (Attention-Deficit/Hyperactivity Disorder, ADHD), mit Schizophrenie assoziierter kognitiver Dysfunktion und kognitiver Dysfunktion ausgewählt ist.

4. Verwendung nach Anspruch 2 oder Anspruch 3 zur Linderung von Gedächtnisverlust oder kognitiven Defiziten.

## Revendications

1. Composition pharmaceutique comprenant une quantité de (*R*)-spiro[1-azabicyclo[2,2,2]octane-3,2'(3'H)-furo[2,3-b]pyridine] comprise entre 0,025 et 0,68 mg par dose unitaire conjointement avec au moins un excipient, diluant ou support pharmaceutiquement acceptable.

2. Utilisation de la (*R*)-spiro[1-azabicyclo[2,2,2]octane-3,2'(3'H)-furo[2,3-b]pyridine] dans la préparation d'un médicament comprenant une dose unitaire dans la gamme de 0,025-0,68 mg pour la thérapie d'un sujet atteint d'une affection neurologique ou psychiatrique.

3. Utilisation selon la revendication 2, **caractérisée en ce que** ladite affection neurologique ou psychiatrique est choisie parmi la maladie d'Alzheimer, le trouble d'hyperactivité avec déficit de l'attention (THADA), le dysfonctionnement cognitif en association avec la schizophrénie ou le dysfonctionnement cognitif.

4. Utilisation selon la revendication 2 ou la revendication 3, pour procurer le soulagement de la perte de mémoire ou de la déficience cognitive.
